# EUROPÄISCHE PATENTSCHRIFT

(11) **EP 1 346 977 B1**
(45) Veröffentlichungstag und Bekanntmachung des Hinweises auf die Patenterteilung: **22.07.2009**
(21) Anmeldenummer: 03004920.9
(22) Anmeldetag: 07.03.2003
(51) Int. Cl.: C07C 213/00, C07C 231/18, C07C 51/347, C07C 67/00, C07C 215/30, C07C 235/34, C07C 59/48, C07C 69/732, C07D 333/20

(54) **Verfahren zur Herstellung von Aryl-aminopropanolen**
Method for producing aryl-aminopropanols
Procédé de préparation d'aryl-aminopropanols

(30) Priorität: 20.03.2002 DE 10212301
(43) Veröffentlichungstag der Anmeldung: 24.09.2003
(73) Patentinhaber: Saltigo GmbH, 40764 Langenfeld (DE)
(72) Erfinder: Eckert, Markus, Dr., Shanghai 200050 (CN); Dreisbach, Claus, Dr., 42799 Leichlingen (DE); Bosch, Boris, Dr., 50668 Köln (DE); Stolle, Andreas, Dr., 42327 Wuppertal (DE)
(74) Vertreter: Wichmann, Birgid

(56) Entgegenhaltungen:
- ASHOK KUMAR ET AL: "A new chemoenzymatic enantioselective synthesis of R-(-)-Tomoxetine, (R)- and (S)-Fluoxetine" TETRAHEDRON LETTERS, ELSEVIER SCIENCE PUBLISHERS, AMSTERDAM, NL, Bd. 32, Nr. 16, 1991, Seiten 1901-1904, XP002121769 ISSN: 0040-4039
- HUANG, H.-L., ET AL: "The synthesis of a chiral fluoxetine intermediate by catalytic enantioselective hydrogenation of benzoylacetamide" TETRAHEDRON: ASYMMETRY, Bd. 9, 1998, Seiten 1637-1640, XP002240972
- SAKURABA S ET AL: "EFFICIENT ASYMMETRIC HYDROGENATION OF BETA- AND GAMMA-AMINO KETONE DERIVATIVES LEADING TO PRACTICAL SYNTHESIS OF FLUOXETINE AND EPROZINOL" CHEMICAL AND PHARMACEUTICAL BULLETIN, PHARMACEUTICAL SOCIETY OF JAPAN. TOKYO, JP, Bd. 43, Nr. 5, 1995, Seiten 748-753, XP001071298 ISSN: 0009-2363
- DEETER J ET AL: "ASYMMETRIC SYNTHESIS AND ABSOLUTE STEREOCHEMISTRY OF LY248686" TETRAHEDRON LETTERS, ELSEVIER SCIENCE PUBLISHERS, AMSTERDAM, NL, Bd. 31, Nr. 49, 26. November 1990 (1990-11-26), Seiten 7101-7104, XP001119089 ISSN: 0040-4039
- LIU H ET AL: "CHEMO-ENZYMATIC SYNTHESIS OF THE ANTIDEPRESSANT DULOXETINE AND ITS ENANTIOMER" CHIRALITY, WILEY-LISS, NEW YORK, US, Bd. 12, Nr. 1, 2000, Seiten 26-29, XP009000316 ISSN: 0899-0042
- LIU, H.-L. ET AL: "Chemoenzymatic synthesis of the non-tricyclic antidepressants Fluoxetine, Tomoxetine and Nisoxetine" J.CHEM.SOC., PERKIN TRANS. 1, 2000, Seiten 1767-1769, XP002240973
- EVERAERE, K. ET AL: "Steric effects in the enentioselective transfer hydrogenation of 2-aroylacetates" TETRAHEDRON:ASYMMETRY, Bd. 10, 1999, Seiten 4663-4666, XP002240974
- BOGER, D.L. ET AL: "Total Synthesis of Bleomycin A2 and Related Agents. 2. Synthesis of (-)-Pyrimidoblamic Acid, epi-(+)-Pyrimidoblamic Acid, (+)-Desacetamidopyrimidoblamic Acid, and (-)-Desacetamidopyrimidoblamic Acid" J.AM.CHEM.SOC., Bd. 116, Nr. 13, 1994, Seiten 5619-5630, XP002240975
- NOYORI, R. ET AL: "Ruthenium(II)-Catalyzed Asymmetric Transfer Hydrogenation of Ketones Using a Formic Acid-Triethylamine Mixture" J.AM.CHEM.SOC., Bd. 118, Nr. 10, 1996, Seiten 2521-2522, XP002240976 -& NOYORI, R. ET AL: J.AM.CHEM.SOC. - SUPPORTING INFORMATION, Bd. 118, 1996, Seiten 1-39, XP002240977

## Beschreibung

Die Erfindung betrifft ein Verfahren zur Herstellung von enantiomerenangereicherten Aryl-aminopropanolen und deren Verwendung sowie Zwischenprodukte.

1-Aryl-3-aminopropan-1-ole haben insbesondere als Zwischenprodukte für die Herstellung von Arzneimitteln technische Bedeutung erlangt. So dienen einige 1-Aryl-3-aminopropan-1-ole beispielsweise als Vorläufersubstanzen für die Herstellung von Serotonin- oder Noradrenalin-Aufnahmeinhibitoren. Bei einigen dieser Inhibitoren konnte nachgewiesen werden, dass bestimmte Enantiomere nicht nur inaktiv oder weniger aktiv sind, sondern sogar unerwünschte Nebenwirkungen aufweisen können (US-A 5,104,899).

Corey und Reichard (Tetrahedron Letters, 39, 5207, 1989) beschreiben ein Verfahren zur Herstellung von S-Fluoxetin, bei dem in einem wichtigen Schritt 3-Chlorpropiophenon mit einem chiralen Boran asymmetrisch zu S-3-Chlor-1-phenyl-1-propanol reduziert wird.

Nach Umsetzung mit Natriumiodid und Methylamin wird dann (S)-3-(Methylamino)-1-phenyl-propan-1-ol erhalten, das weiter zum Endprodukt umgesetzt werden kann. Nachteilig an diesem Verfahren ist, dass teure Reagentien eingesetzt werden müssen und die Gesamtausbeute bei lediglich 77 bis 82 % liegt.

Ein Verfahren zur Herstellung von enantiomerenangereichertem (1S)-3-(Methylamino)-1-(2-thiophen-yl)-1-propanol ausgehend von 1-(2-Thiophen-yl)-3-chlorpropan-1-on wird in Chirality 2000, 12, 26-29 beschrieben. Nach der Reduktion zum racemischen 3-Chlor-1-(2-thienyl)-1-propanol wird das Racemat enzymatisch getrennt und das (S)-Enantiomer weiter mit NaI und Methylamin zu (S)-3-(Methylamino)-1-(2-thiophen-yl)-propan-1-ol umgesetzt. Diese Methode hat den Nachteil, dass bei enzymatischen Racemattrennungen prinzipiell nur 50 % des gewünschten Enantiomers erhalten werden können und die Gesamtausbeute daher wirtschaftlich nicht akzeptabel ist.

Eine ähnliche Syntheseroute wird in J. Lab. Comp. Radiopharm. 1995, 36, 213-223 beschrieben, in der 1-(2-Thiophen-yl)-3-chlorpropan-1-on mit Boran und einem Oxazaborolidin asymmetrisch reduziert wird. Die Ausbeute in diesem Schritt beträgt nur 61 %, was das Gesamtverfahren unwirtschaftlich macht.

Es bestand daher das Bedürfnis, ein effizientes und breit anwendbares Verfahren zur Herstellung von enantiomerenangereicherten Aryl-aminopropanolen bereitzustellen, das von einfach erhältlichen Edukten ausgeht.

Es wurde nun ein Verfahren zur Herstellung von enantiomerenangereicherten Verbindungen der Formel (I) gefunden

Ar-CH(OH)-CH₂-CH₂-NR¹R² (I),

in der
- Ar: für einen substituierten oder unsubstituierten 2-Thiophen-yl- oder 3-Thiophen-ylrest und
- R¹ und R²: jeweils unabhängig voneinander für Wasserstoff, C₁-C₂₀-Alkyl, C₄-C₁₄-Aryl oder C₅-C₁₅-Arylalkyl stehen oder die beiden Reste R¹ und R² zusammen für C₃-C₁₂-Alkylen stehen,
das dadurch gekennzeichnet ist, dass
a) Verbindungen der Formel (II) in enantiomerenangereicherte Verbindungen der Formel (III) oder Verbindungen der Formel (IV) in enantiomerenangereicherte Verbindungen der Formel (V) überführt werden

   Ar-CO-CH₂-COOR³ (II)

   Ar-CH(OH)-CH₂-COOR³ (III)

   Ar-CO-CH₂-CONR¹R² (IV)

   Ar-CH(OH)-CH₂-CONR¹R² (V),

   wobei jeweils
   Ar die unter der Formel (I) genannte Bedeutung besitzt und
   R¹ und R² die unter der Formel (I) genannte Bedeutung besitzen und
   R³ für Wasserstoff, C₁-C₂₀-Alkyl, C₄-C₁₄-Aryl oder C₅-C₁₅-Arylalkyl steht,
   und wobei die Umsetzung
   i) in Gegenwart eines Rutheniumkomplexe enthaltenden Katalysators, wobei die Rutheniumkomplexe durch Umsetzung von Verbindungen der Formel (XII) mit Verbindungen der Formel (XIII) erhältlich sind, oder Komplexe der Formel (XIV), wobei in den Verbindungen der Formel (XII)

      [RuX₂(Aren)]₂ (XII)

      Aren steht für eine koordinierte aromatische Verbindung mit 6 bis 12 Ringkohlenstoffatomen, die weiterhin mit bis zu 6 Resten substituiert sein kann, die jeweils unabhängig voneinander ausgewählt sind aus der Gruppe C₁-C₈-Alkyl, Benzyl und Phenyl und
      X für Chlor, Brom oder Iod steht,
      und wobei in der Formel (XIII) 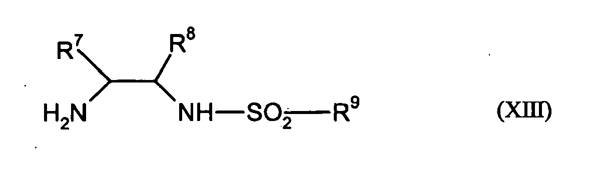 R⁷ und R jeweils unabhängig voneinander beispielsweise für C₁-C₂₀-Alkyl, C₄-C₁₅-Aryl oder C₅-C₁₆-Awlalkyl oder R und R zusammen für einen geradkettigen oder verzweigten C₃-C₁₂-Alkylen-Rest stehen und
      R⁹ für C₁-C₂₀-Alkyl, C₁-C₂₀-Fluoralkyl oder C₄-C₁₅-Aryl steht,
      und wobei in der Formel (XIV)

      [RuX₂(aren) {(XIII)}] (XIV)

      Aren und X jeweils die unter Formel (XII) angegebene Bedeutung besitzen und (XIII) für Verbindungen der Formel (XIII) mit der dort angegebenen Bedeutung steht,
   ii) in Gegenwart von mindestens einem Amin, das zumindest teilweise in protonierter Form vorliegt,
   iii) mit Ameisensäure, Formiaten oder Mischungen davon
      erfolgt und
b) für den Fall, dass für Schritt a) Verbindungen der Formel (II) eingesetzt wurden, die enantiomerenangereicherten Verbindungen der Formel (III) mit Aminen der Formel (VI)

   HNR¹R² (VI),

   in der R¹ und R² die unter der Formel (I) genannte Bedeutung besitzen,
   zu enantiomerenangereicherten Verbindungen der Formel (V) mit der oben genannten Bedeutung umgesetzt werden und
c) die enantiomerenangereicherten Verbindungen der Formel (V) durch Reduktion in enantiomerenangereicherte Verbindungen der Formel (I) mit der oben genannten Bedeutung überführt werden.

Besonders bevorzugt steht Ar für einen 2-Thiophen-ylrest.

Es sei darauf hingewiesen, dass vom Umfang der Erfindung auch beliebige Kombinationen der genannten Bereiche und Vorzugsbereiche für jedes Merkmal umfasst sind.

Enantiomerenangereichert im Sinne der Erfindung bedeutet enantiomerenreine Verbindungen oder Mischungen von Enantiomeren einer Verbindung, in denen ein Enantiomer in einem Enantiomerenüberschuss, im Folgenden auch ee (enantiomeric excess) genannt, im Vergleich zum anderen Enantiomer vorliegt. Bevorzugt beträgt dieser Enantiomerenüberschuss 10 bis 100 % ee, besonders bevorzugt 60 bis 100 % ee und ganz besonders bevorzugt 85 bis 100 % ee.

Enantiomerenangereichert im Sinne der Erfindung bezieht sich insbesondere auf die Konfiguration des Kohlenstoffatoms, das dem Rest Ar benachbart ist.

Bevorzugt stehen in den Formeln (I), (IV), (V) und (VI) R¹ und R² jeweils unabhängig für Wasserstoff, Methyl, Ethyl, Isopropyl, Phenyl oder Benzyl.

Besonders bevorzugt steht in den Formeln (I), (IV) und (V) NR¹R² als Ganzes für Methylamino, Ethylamino und Isopropylamino, beziehungsweise in Formel (VI) HNR¹R² für Methylamin, Ethylamin und Isopropylamin.

Ganz besonders bevorzugt steht in den Formeln (I), (IV) und (V) NR¹R² als Ganzes für Methylamino beziehungsweise in Formel (VI) HNR¹R² für Methylamin.

Bevorzugt steht in den Formeln (II) und (III) R³ jeweils für Wasserstoff, Methyl, Ethyl, Isopropyl, n-Propyl, tert.-Butyl, n-Butyl, iso-Butyl, Phenyl oder Benzyl, wobei Methyl und Ethyl besonders bevorzugt sind und Methyl ganz besonders bevorzugt ist.

Für das erfindungsgemäße Verfahren bevorzugte Verbindungen der Formel (II) sind:

Methyl-3-oxo-3-(2-thiophen-yl)propanoat, Ethyl-3-oxo-3-(2-thiophen-yl)propanoat,

Isopropyl-3-oxo-3-(2-thiophen-yl)propanoat, tert.-Butyl-3-oxo-3-(2-thiophen-yl)propanoat, 2-Ethylhexyl-3-oxo-3-(2-thiophen-yl)propanoat.

Für das erfindungsgemäße Verfahren bevorzugte Verbindungen der Formel (IV) sind:

3-Oxo-3-(2-thiophen-yl)propanamid, N-Methyl-3-oxo-3-(2-thiophen-yl)-propanamid und N-Benzyl-3-oxo-3-(2-thiophen-yl)propanamid.

Für das erfindungsgemäße Verfahren bevorzugt ist der Einsatz von Verbindungen der Formel (II) in Schritt a) mit den oben genannten Bedeutungen und Vorzugsbereichen.

Für das erfindungsgemäße Verfahren besonders bevorzugt ist der Einsatz von Verbindungen der Formel (II) in Schritt a) mit den oben genannten Bedeutungen und Vorzugsbereichen für Ar und R¹ und in Schritt b) die Umsetzung mit Methylamin.

Die für das erfindungsgemäße Verfahren einsetzbaren Verbindungen der Formel (IV) können analog zu den Verbindungen der Formel (II) beispielsweise durch basenkatalysierte Umsetzung von Verbindungen der Formel (IX) mit Verbindungen der Formel (XI)

R³-O-CO-NR¹R² (XI)

erhalten werden,
in denen die Reste R¹, R² und R³ jeweils unabhängig voneinander, vorzugsweise identisch die gleiche Bedeutung und Vorzugsbereiche besitzen, die unter den Formeln (II) und (IV) angegeben wurden.

Beispielsweise seien als Verbindungen der Formel (XI) genannt:
N-Methyl-methylcarbamat, N-Methyl-ethylcarbamat, N-Methyl-methylcarbamat, N-Methyl-ethylcarbamat, N,N-Dimethyl-methylcarbamat und N,N-Dimethyl-ethylcarbamat.

Eine solche Umsetzung wird z.B. in Tetrahedron Lett. 1998, 39, 4995 beschrieben und kann beispielweise analog für die Umsetzung von 2-Acetylthiophen mit N-Methyl-methylcarbamat oder N-Methyl-ethylcarbamat angewandt werden.

Die für das erfindungsgemäße Verfahren einsetzbaren Verbindungen der Formel (IV) können ebenso über eine basenkatalysierte Umsetzung der Verbindungen der allgemeinen Formel (IX) mit Isocyanaten erhalten werden, wie in Synth. Commun. 1987, 17, 13-18 beschrieben. Als Beispiel sei die Umsetzung von 2-Acetylthiophen mit Methylisocyanat, Ethylisocyanat oder Benzylisocyanat genannt.

Aryl steht im Rahmen der Erfindung beispielsweise und bevorzugt für carbocyclische aromatische Reste oder heteroaromatische Reste, die keines, ein, zwei oder drei Heteroatome pro Cyclus, im gesamten heteroaromatische Rest mindestens jedoch ein Heteroatom enthalten, das ausgewählt ist aus der Gruppe Stickstoff, Schwefel oder Sauerstoff. Weiterhin können die carbocyclischen aromatischen Reste oder heteroaromatischen Reste mit bis zu fünf Substituenten pro Cyclus substituiert sein, die jeweils unabhängig voneinander beispielsweise und bevorzugt ausgewählt sind aus der Gruppe Hydroxy, C₁-C₁₂-Alkyl, Cyano, COOH, COOM, wobei M für ein Alkalimetallion oder ein halbes Äquivalent eines Erdalkalimetallions steht, COO-(C₁-C₁₂-Alkyl), COO-(C₄-C₁₀-Aryl), CO-(C₁-C₁₂-Alkyl), CO-(C₄-C₁₀-Aryl), O-(C₁-C₁₂-Alkyl), O-(C₄-C₁₀)-Aryl, N(C₁-C₁₂-Alkyl)₂, NH-(C₁-C₁₂-Alkyl), Fluor, Chlor, Brom, C₁-C₁₂-Fluoralkyl, wobei Fluoralkyl für einen einfach, mehrfach oder vollständig durch Fluor substituierten Alkylrest gemäß obiger Definition steht, CONH2, CONH-(C₁-C₁₂-Alkyl), NHCOO-(C₁-C₁₂-Alkyl). Gleiches gilt für den Arylteil eines Arylalkyl-Restes.

Alkyl bzw. Alkylen bedeutet im Rahmen der Erfindung jeweils unabhängig einen geradkettigen, cyclischen, verzweigten oder unverzweigten Alkyl- bzw. Alkylenrest, der gegebenenfalls durch C₁-C₄-Alkoxy-Reste weiter substituiert sein kann. Gleiches gilt für den Alkylenteil eines Arylalkyl-Restes.

Beispielsweise steht in allen Zusammenhängen bevorzugt C₁-C₆-Alkyl für Methyl, Ethyl, n-Propyl, Isopropyl, n-Butyl, tert.-Butyl, n-Pentyl, Cyclohexyl und n-Hexyl, C₁-C₈-Alkyl darüber hinaus beispielsweise für n-Heptyl, n-Octyl oder iso-Octyl, C₁-C₁₂-Alkyl, weiter darüber hinaus z.B. für n-Decyl und n-Dodecyl und C₁-C₂₀-Alkyl noch weiter darüber hinaus für n-Hexadecyl und n-Octadecyl.

Beispielsweise steht C₁-C₄-Alkylen in allen Zusammenhängen bevorzugt für Methylen, 1,1-Ethylen, 1,2-Ethylen, 1,1-Propylen, 1,2-Propylen, 1,3-Propylen, 1,1-Butylen, 1,2-Butylen, 2,3-Butylen und 1,4-Butylen, C₁-C₈-Alkylen darüber hinaus für 1,5-Pentylen, 1,6-Hexylen, 1,1-Cyclohexylen, 1,4-Cyclohexylen, 1,2-Cyclohexylen und 1,8-Octylen.

Die allgemeine Bezeichnung Aryl als Substituent umfasst carbocyclische Reste und heteroaromatische Reste, in denen keines, ein, zwei oder drei Gerüstatome pro Cyclus, im gesamten Rest mindestens jedoch ein Gerüstatom Heteroatome ausgewählt aus der Gruppe Stickstoff, Schwefel oder Sauerstoff sind. C₅-C₁₀-Aryl steht beispielsweise und bevorzugt für Phenyl, Pyridyl, o-,m-, oder p-Tolyl, C₅-C₁₄-Aryl darüber hinaus für Anthracenyl.

Gleiches gilt für den Arylteil eines Arylalkyl-Restes. C₆-C₁₅-Arylalkyl steht beispielsweise und bevorzugt für Benzyl.

**Fluoralkyl** bedeutet im Rahmen der Erfindung jeweils unabhängig einen geradkettigen, cyclischen, verzweigten oder unverzweigten Alkylrest, der mit einem, mehreren oder vollständig mit Fluoratomen substituiert sein können.

Beispielsweise und bevorzugt steht C₁-C₈-Fluoralkyl in allen Zusammenhängen bevorzugt für Trifluormethyl, Trichlormethyl, 2,2,2-Trifluorethyl, Pentafluorethyl und Nonafluorbutyl, C₁-C₈-Fluoralkyl für Trifluormethyl, 2,2,2-Trifluorethyl, Pentafluorethyl und Nonafluorbutyl.

Im Schritt a) des erfindungsgemäßen Verfahrens werden in Gegenwart eines Übergangsmetallkatalysators die Verbindungen der Formel (II) in enantiomerenangereicherte Verbindungen der Formel (III) oder Verbindungen der Formel (IV) in enantiomerenangereicherte Verbindungen der Formel (V) überführt, wobei die Umsetzung
iii) in Gegenwart eines Rutheniumkomplexe enthaltenden Katalysators und
iv) in Gegenwart von mindestens einem Amin, das zumindest teilweise in protonierter Form vorliegt,
iii) mit Ameisensäure, Formiaten oder Mischungen davon
erfolgt.

Die katalytische Reduktion von Ketonen zu enantiomerenangereicherten sekundären Alkoholen ist prinzipiell bekannt. Als Reduktionsmittel kommen dabei üblicherweise molekularer Wasserstoff oder bei sogenannten Transferhydrierungen Wasserstoff übertragende Verbindungen wie zum Beispiel Ameisensäure oder Isopropanol zum Einsatz.

Beispielsweise wird die asymmetrische Hydrierung von Aryl-β-ketoestern mit Ru-Phosphin-Katalysatoren in Tetrahedron 1995, 27, 4801 oder Org. and Organomet. Synth. 1999, 2, 175 beschrieben. Ein Übersichtsartikel findet sich in Tetrahedron Asymmetry 1997, 8, 3327. Insbesondere eignen sich Rutheniumkatalysatoren mit Binaphthyl- oder Biphenylphosphinliganden für die asymmetrische Hydrierung, wie in EP 529 444, EP 643 065, EP 749 973 und EP 764 652 beschrieben.

Es werden solche Katalysatoren verwendet, die Rutheniumkomplexe enthalten. Rutheniumkomplexe sind solche, die durch Umsetzung von Verbindungen der Formel (XII) mit Verbindungen der Formel (XIII) erhältlich sind, oder Komplexe der Formel (XIV). Besonders bevorzugt werden solche Rutheniumkomplexe eingesetzt, die durch Umsetzung von Verbindungen der Formel (XII) mit Verbindungen der Formel (XIII) erhältlich sind. In einer bevorzugten Ausführungsform beträgt dabei das molare Verhältnis von Verbindungen der Formel (XIII) und Verbindungen der Formel (II) 2:1 bis 3:1, besonders bevorzugt 2,01:1 bis 2,4:1.

Vorteilhafterweise werden Verbindungen der Formel (XIII) und Verbindungen der Formel (XII) gemischt und die Mischung in organischem Lösungsmittel aufgenommen. Die resultierende Mischung kann weiterhin vorteilhafterweise vor Zugabe zur Reaktionsmischung mit einer Base, bevorzugt einem tertiären Amin, versetzt und beispielsweise und bevorzugt 10 bis 30 min gerührt werden, wobei die molare Menge tertiären Amins beispielsweise und bevorzugt 1:1 bis 3:1, besonders bevorzugt 1:1 bis 2:1 bezogen auf Verbindungen der Formel (XIII) beträgt.

Für organische Lösungsmittel und Amine gelten die gleichen Angaben und Vorzugsbereiche, die weiter unten detailliert beschrieben werden.

In den Verbindungen der Formel (XII)

[RuX₂(Aren)]₂ (XII)

steht
- Aren: für eine koordinierte aromatische Verbindung mit 6 bis 12 Ringkohlenstoffatomen, die weiterhin mit bis zu 6 Resten substituiert sein kann, die jeweils unabhängig voneinander ausgewählt sind aus der Gruppe C₁-C₈-Alkyl, Benzyl und Phenyl, und
- X: für Chlor, Brom oder Iod, besonders bevorzugt für Chlor.

Bevorzugt steht Aren für Benzol oder Naphthalin, das mit bis zu 6 Resten substituiert sein kann, die jeweils unabhängig voneinander ausgewählt sind aus der Gruppe Methyl, Ethyl, n-Propyl, Isopropyl und tert.-Butyl.

Bevorzugt steht Aren für Mesitylen, Cumol oder Benzol.

Besonders bevorzugte Verbindungen der Formel (XII) sind:

Benzoldichlororuthenium-Dimer, Mesitylendichlororuthenium-Dimer und Cumoldichlororuthenium-Dimer, wobei Cumoldichlororuthenium-Dimer noch weiter bevorzugt ist.

In der Formel (XIII) 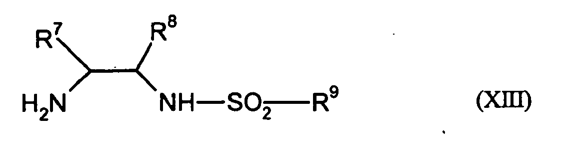 stehen
R⁷ und R⁸ jeweils unabhängig voneinander für C₁-C₂₀-Alkyl, C₄-C₁₅-Aryl oder C₅-C₁₆-Arylalkyl oder R und R⁸ zusammen für einen geradkettigen oder verzweigten C₃-C₁₂-Alkylen-Rest und
R⁹ für C₁-C₂₀-Alkyl, C₁-C₂₀-Fluoralkyl oder C₄-C₁₅-Aryl.

Bevorzugt stehen R⁷ und R⁸ jeweils identisch für Phenyl oder zusammen für geradkettiges C₃-C₈-Alkylen wie zum Beispiel 1,3-Propylen oder 1,4-Butylen, besonders bevorzugt stehen R⁷ und R⁸ jeweils identisch für Phenyl.
- R⁹: steht bevorzugt für C₁-C₄-Alkyl, C₁-C₄-Fluoralkyl, Phenyl oder Naphthyl, das mit keinem, einem, zwei, drei vier oder fünf Resten weiter substituiert sein kann, die ausgewählt sind aus der Gruppe C₁-C₄-Alkyl, C₁-C₄-Alkoxy, C₁-C₄-Fluoralkyl, Fluor und Chlor.
- R⁹: steht besonders bevorzugt für Methyl, Trifluormethyl, Pentafluorethyl, Nonafluorbutyl, Phenyl, p-Tolyl, p-Ethylphenyl, p-Anisyl, p-Ethoxyphenyl, p-Chlorphenyl, 2,4,6-Trimethylphenyl, 2,4,6-Triisopropylphenyl, p-Fluorphenyl, Pentafluorphenyl und Naphthyl.
- R⁹: steht ganz besonders bevorzugt für p-Tolyl, Phenyl, Naphtyl.
- R⁹: steht noch weiter bevorzugt für p-Tolyl.

Bevorzugt weisen die Verbindungen der Formel (XIII) eine Stereoisomerenreinheit von 90 % oder mehr, besonders bevorzugt von 95 % oder mehr und ganz besonders bevorzugt von 98,5 % oder mehr auf.

Als Verbindungen der Formel (XIII) seien genannt:
N-[(1R,2R) und (1S,2S)-2-amino-1,2-diphenylethyl]-p-tolylsulfonamid, N-[(1R,2R) und (1S,2S)-2-amino-1,2-diphenylethyl]-o-tolylsulfonamid, N-[(1R,2R) und (1S,2S)-2-amino-1,2-diphenylethyl]-m-tolylsulfonamid, N-[(1R,2R) und (1S,2S)-2-amino-1,2-diphenylethyl]-phenylsulfonamid, N-[(1R,2R) und (1S,2S)-2-amino-1,2-diphenylethyl]-4-ethylphenylsulfonamid, N-[(1R,2R) und (1S,2S)-2-amino-1,2-diphenylethyl]-3-ethylphenylsulfonamid, N-[(1R,2R) und (1S,2S)-2-amino-1,2-diphenylethyl]-2-ethylphenylsulfonamid, N-[(1R,2R) und (1S,2S)-2-amino-1,2-diphenylethyl]-2,4,6-trimethylphenylsulfonamid, N-[(1R,2R) und (1S,2S)-2-amino-1,2-diphenylethyl]-2,4,6-triisopropylphenylsulfonamid, N-[(1R,2R) und (1S,2S)-2-amino-1,2-diphenylethyl]-4-chlorphenylsulfonamid, N-[(1R,2R) und (1S,2S)-2-amino-1,2-diphenylethyl]-3-chlorphenylsulfonamid, N-[(1R,2R) und (1S,2S)-2-amino-1,2-diphenylethyl]-2-chlorphenylsulfonamid, N-[(1R,2R) und (1S,2S)-2-amino-1,2-diphenylethyl]-4-fluorphenylsulfonamid, N-[(1R,2R) und (1S,2S)-2-amino-1,2-diphenylethyl]-3-fluorphenylsulfonamid, N-[(1R,2R) und (1S,2S)-2-amino-1,2-diphenylethyl]-2-fluorphenylsulfonamid, N-[(1R,2R) und (1S,2S)-2-amino-1,2-diphenylethyl]-4-methoxyphenylsulfonamid, N-[(1R,2R) und (1S,2S)-2-amino-1,2-diphenylethyl]-3-methoxyphenylsulfonamid, N-[(1R,2R) und (1S,2S)-2-amino-1,2-diphenylethyl]-2-methoxyphenylsulfonamid, N-[(1R,2R) und (1S,2S)-2-amino-1,2-diphenylethyl]-1-naphtylsulfonamid, N-[(1R,2R) und (1S,2S)-2-amino-1,2-diphenylethyl]-2-naphtylsulfonamid, N-[(1R,2R) und (1S,2S)-2-amino-1,2-diphenylethyl]-pentafluorphenylsulfonamid, N-[(1R,2R) und (1S,2S)-2-amino-1,2-diphenylethyl]-methansulfonamid, N-[(1R,2R) und (1S,2S)-2-amino-1,2-diphenylethyl]-trifluormethansulfonamid, N-[(1R,2R) und (1S,2S)-2-aminocyclohexyl]-p-tolylsulfonamid, N-[(1R,2R) und (1S,2S)-2-aminocyclohexyl]-o-tolylsulfonamid, N-[(1R,2R) und (1S,2S)-2-aminocyclohexyl]-m-tolylsulfonamid, N-[(1R,2R) und (1S,2S)-2-aminocyclohexyl]-phenylsulfonamid, N-[(1R,2R) und (1S,2S)-2-aminocyclohexyl]-4-ethylphenylsulfonamid, N-[(1R,2R) und (1S,2S)-2-aminocyclohexyl]-3-ethylphenylsulfonamid, N-[(1R,2R) und (1S,2S)-2-aminocyclohexyl]-2-ethylphenylsulfonamid, N-[(1R,2R) und (1S,2S)-2-aminocyclohexyl]-2,4,6-trimethylphenylsulfonamid, N-[(1R,2R) und (1S,2S)-2-aminocyclohexyl]-2,4,6-triisopropylphenylsulfonamid, N-[(1R,2R) und (1S,2S)-2-aminocyclohexyl]-4-chlorphenylsulfonamid, N-[(1R,2R) und (1S,2S)-2-aminocyclohexyl]-3-chlorphenylsulfonamid, N-[(1R,2R) und (1S,2S)-2-aminocyclohexyl]-2-chlorphenylsulfonamid, N-[(1R,2R) und (1S,2S)-2-aminocyclohexyl]-4-fluorphenylsulfonamid, N-[(1R,2R) und (1S,2S)-2-aminocyclohexyl]-3-fluorphenylsulfonamid, N-[(1R,2R) und (1S,2S)-2-aminocyclohexyl]-2-fluorphenylsulfonamid, N-[(1R,2R) und (1S,2S)-2-aminocyclohexyl]-4-methoxyphenylsulfonamid, N-[(1R,2R) und (1S,2S)-2-aminocyclohexyl]-3-methoxyphenylsulfonamid, N-[(1R,2R) und (1S,2S)-2-aminocyclohexyl]-2-methoxyphenylsulfonamid, N-[(1R,2R) und (1S,2S)-2-aminocyclohexyl]-1-naphtylsulfonamid, N-[(1R,2R) und (1S,2S)-2-aminocyclohexyl]-2-naphtylsulfonamid, N-[(1R,2R) und (1S,2S)-2-aminocyclohexyl]-pentafluorphenylsulfonamid, N-[(1R,2R) und (1S,2S)-2-aminocyclohexyl]-methansulfonamid, N-[(1R,2R) und (1S,2S)-2-aminocyclohexyl]-trifluormethansulfonamid, N-[(1R,2R) und (1S,2S)-2-aminocyclopentyl]-p-tolylsulfonamid, N-[(1R,2R) und (1S,2S)-2-aminocyclopentyl]-o-tolylsulfonamid, N-[(1R,2R) und (1S,2S)-2-aminocyclopentyl]-m-tolylsulfonamid, N-[(1R,2R) und (1S,2S)-2-aminocyclopentyl]-phenylsulfonamid, N-[(1R,2R) und (1S,2S)-2-aminocyclopentyl]-4-ethylphenylsulfonamid, N-[(1R,2R) und (1S,2S)-2-aminocyclopentyl]-3-ethylphenylsulfonamid, N-[(1R,2R) und (1S,2S)-2-aminocyclopentyl]-2-ethylphenylsulfonamid, N-[(1R,2R) und (1S,2S)-2-aminocyclopentyl]-2,4,6-trimethylphenylsulfonamid, N-[(1R,2R) und (1S,2S)-2-aminocyclopentyl]-2,4,6-triisopropylphenylsulfonamid, N-[(1R,2R) und (1S,2S)-2-aminocyclopentyl]-4-chlorphenylsulfonamid, N-[(1R,2R) und (1S,2S)-2-aminocyclopentyl]-3-chlorphenylsulfonamid, N-[(1R,2R) und (1S,2S)-2-aminocyclopentyl]-2-chlorphenylsulfonamid, N-[(1R,2R) und (1S,2S)-2-aminocyclopentyl]-4-fluorphenylsulfonamid, N-[(1R,2R) und (1S,2S)-2-aminocyclopentyl]-3-fluorphenylsulfonamid, N-[(1R,2R) und (1S,2S)-2-aminocyclopentyl]-2-fluorphenylsulfonamid, N-[(1R,2R) und (1S,2S)-2-aminocyclopentyl]-4-methoxyphenylsulfonamid, N-[(1R,2R) und (1S,2S)-2-aminocyclopentyl]-3-methoxyphenylsulfonamid, N-[(1R,2R) und (1S,2S)-2-aminocyclopentyl]-2-methoxyphenylsulfonamid, N-[(1R,2R) und (1S,2S)-2-aminocyclopentyl]-1-naphtylsulfonamid, N-[(1R,2R) und (1S,2S)-2-aminocyclopentyl]-2-naphtylsulfonamid, N-[(1R,2R) und (1S,2S)-2-aminocyclopentyl]-pentafluorphenylsulfonamid, N-[(1R,2R) und (1S,2S)-2-aminocyclopentyl]-methansulfonamid, N-[(1R,2R) und (1S,2S)-2-aminocyclopentyl]-trifluormethansulfonamid.

In der Formel (XIV)

[RuX₂(aren) {(XIII)}] (XIV)

besitzen Aren und X jeweils die unter Formel (XII) angegebene Bedeutung und Vorzugsbereiche, (XIII) steht in der Formel (XIV) für Verbindungen der Formel (XIII) mit den dort angegebenen Bedeutung und Vorzugsbereichen.

Als Verbindungen der Formel (XIV) seien genannt:
[N-[(1R,2R und 1S,2S)-2-(amino-κN)-1,2-diphenylethyl]-p-tolylsulfonamidato-κN]chloro-[(η⁶)-cumol]-ruthenium(II)
[N-[(1R,2R und 1S,2S)-2-(amino-κN)-1,2-diphenylethyl]-o-tolylsulfonamidato-κN]-chloro[(η⁶)-cumol]-ruthenium(II)
[N-[(1R,2R und 1S,2S)-2-(amino-κN)-1,2-diphenylethyl]-m-tolylsulfonamidato-κN]-chloro[(η⁶)-cumol]-ruthenium(II)
[N-[(1R,2R und 1S,2S)-2-(amino-κN)-1,2-diphenylethyl]-phenylsulfonamidato-κN]-chloro [(η⁶)-cumol]-ruthenium(II)
[N-[(1R,2R und 1S,2S)-2-(amino-κN)-1,2-diphenylethyl]-4-ethylphenylsulfonamidato-κN]chloror(η⁶)-cumol]-ruthenium(II)
[N-[(1R,2R und 1S,2S)-2-(amino-κN)-1,2-diphenylethyl]-3-ethylphenylsulfonamidato-κN]chloro[(η⁶)-cumol]-ruthenium(II)
[N-[(1R,2R und 1S,2S)-2-(amino-κN)-1,2-diphenylethyl]-2-ethylphenylsulfonamidato-κN]chloro[(η⁶)-cumol]-ruthenium(II)
[N-[(1R,2R und 1S,2S)-2-(amino-κN)-1,2-diphenylethyl]-2,4,6-trimethylphenylsulfon-amidato-κN]chloro[(η⁶)-cumol]-ruthenium(II)
[N-[(1R,2R und 1S,2S)-2-(amino-κN)-1,2-diphenylethyl]-2,4,6-triisopropylphenylsulfonamidato-κN]chloro[(η⁶)-cumol]-ruthenium(II)
[N-[(1R,2R und 1S,2S)-2-(amino-κN)-1,2-diphenylethyl]-4-chlorphenylsulfonamidato-κN]chloro[(η⁶)-cumol]-ruthenium(II)
[N-[(1R,2R und 1S,2S)-2-(amino-κN)-1,2-diphenylethyl]-3-chlorphenylsulfonamidato-κN]chloro[(η⁶)-cumol]-ruthenium(II)
[N-[(1R,2R und 1S,2S)-2-(amino-κN)-1,2-diphenylethyl]-2-chlorphenylsulfonamidato-κN]chloro[(η⁶)-cumol]-ruthenium(II)
[N-[(1R,2R und 1S,2S)-2-(amino-κN)-1,2-diphenylethyl]-4-fluorphenylsulfonamidato-κN]chloro[(η⁶)-cumol]-ruthenium(II)
[N-[(1R,2R und 1S,2S)-2-(amino-κN)-1,2-diphenylethyl]-3-fluorphenylsulfonamidato-κN]chloro[(η⁶)-cumol]-ruthenium(II)
[N-[(1R,2R und 1S,2S)-2-(amino-κN)-1,2-diphenylethyl]-2-fluorphenylsulfonamidato-κN]chloro[(η⁶)-cumol]-ruthenium(II)
[N-[(1R,2R und 1S,2S)-2-(amino-κN)-1,2-diphenylethyl]-4-methoxyphenylsulfonamidato-κN]chloro[(η⁶)-cumol]-ruthenium(II)
[N-[(1R,2R und 1S,2S)-2-(amino-κN)-1,2-diphenylethyl]-3-methoxyphenylsulfonamidato-κN]chloro[(η⁶)-cumol]-ruthenium(II)
[N-[(1R,2R und 1S,2S)-2-(amino-κN)-1,2-diphenylethyl]-2-methoxyphenylsulfonamidato-κN]chloro[(η⁶)-cumol]-ruthenium(II)ato-κN]chloro[(η⁶)-cumol]-ruthenium(II)
[N-[(1R,2R und 1S,2S)-2-(amino-κN)-1,2-diphenylethyl]-1-naphtylsulfonamidato-κN]-chloro[(η⁶)-cumol]-ruthenium(II)
[N-[(1R,2R und 1S,2S)-2-(amino-κN)-1,2-diphenylethyl]-2-naphtylsulfonamidato-κN]-chloro[(η⁶)-cumol]-ruthenium(II)
[N-[(1R,2R und 1S,2S)-2-(amino-κN)-1,2-diphenylethyl]-pentafluorphenylsulfonamidato-κN]chloro[(η⁶)-cumol]-ruthenium(II)
[N-[(1R,2R und 1S,2S)-2-(amino-κN)-1,2-diphenylethyl]-methansulfonamidato-κN]-chloro[(η⁶)-cumol]-ruthenium(II)
[N-[(1R,2R und 1S,2S)-2-(amino-κN)-1,2-diphenylethyl]-trifluormethansulfonamidato-κN]chloro[(η⁶)-cumol]-ruthenium(II)
[N-[(1R,2R und 1S,2S)-2-(amino-κN)-1,2-diphenylethyl]-p-tolylsulfonamidato-κN]-chloro[(η⁶)-1,3,5-trimethylbenzol]-ruthenium(II)
[N-[(1R,2R und 1S,2S)-2-(amino-κN)-1,2-diphenylethyl]-o-tolylsulfonamidato-κN]-chloro[(η⁶)-1,3,5-trimethylbenzol]-ruthenium(II)
[N-[(1R,2R und 1S,2S)-2-(amino-κN)-1,2-diphenylethyl]-m-tolylsulfonamidato-κN]-chloro[(η⁶)-1,3,5-trimethylbenzol]-ruthenium(II)
[N-[(1R,2R und 1S,2S)-2-(amino-κN)-1,2-diphenylethyl]-phenylsulfonamidato-κN]-chloro[(η⁶)-1,3,5-trimethylbenzol]-ruthenium(II)
[N-[(1R,2R und 1S,2S)-2-(amino-κN)-1,2-diphenylethyl]-4-ethylphenylsulfonamidato-κN]chloro[(η⁶)-1,3,5-trimethylbenzol]-ruthenium(II)
[N-[(1R,2R und 1S,2S)-2-(amino-κN)-1,2-diphenylethyl]-3-ethylphenylsulfonamidato-κN]chloro[(η⁶)-1,3,5-trimethylbenzol]-ruthenium(II)
[N-[(1R,2R und 1S,2S)-2-(amino-κN)-1,2-diphenylethyl]-2-ethylphenylsulfonamidato-κN]chloro[(η⁶)-1,3,5-trimethylbenzol]-ruthenium(II)
[N-[(1R,2R und 1S,2S)-2-(amino-κN)-1,2-diphenylethyl]-2,4,6-trimethylphenylsulfonamidato-κN]chloro[(η⁶)-1,3,5-trimethylbenzol]-ruthenium(II)
[N-[(1R,2R und 1S,2S)-2-(amino-κN)-1,2-diphenylethyl]-2,4,6-triisopropylphenyl-sulfonamidato-κN]chloro[(η⁶)-1,3,5-trimethylbenzol]-ruthenium(II)
[N-[(1R,2R und 1S,2S)-2-(amino-κN)-1,2-diphenylethyl]-4-chlorphenylsulfonamidato-κN]chloro[(η⁶)-1,3,5-trimethylbenzol]-ruthenium(II)
[N-[(1R,2R und 1S,2S)-2-(amino-κN)-1,2-diphenylethyl]-3-chlorphenylsulfonamidato-κN]chloro[(η⁶)-1,3,5-trimethylbenzol]-ruthenium(II)
[N-[(1R,2R und 1S,2S)-2-(amino-κN)-1,2-diphenylethyl]-2-chlorphenylsulfonamidato-κN]chloro[(η⁶)-1,3,5-trimethylbenzol]-ruthenium(II)
[N-[(1R,2R und 1S,2S)-2-(amino-κN)-1,2-diphenylethyl]-4-fluorphenylsulfonamidato-κN]chloro[(η⁶)-1,3,5-trimethylbenzol]-ruthenium(II)
[N-[(1R,2R und 1S,2S)-2-(amino-κN)-1,2-diphenytethyl]-3-fluorphenylsulfonamidato-κN]chloro[(η⁶)-1,3,5-trimethylbenzol]-ruthenium(II)
[N-[(1R,2R und 1S,2S)-2-(amino-κN)-1,2-diphenylethyl]-2-fluorphenylsulfonamidato-κN]chloro[(η⁶)-1,3,5-trimethylbenzol]-ruthenium(II)
[N-[(1R,2R und 1S,2S)-2-(amino-κN)-1,2-diphenylethyl]-4-methoxyphenylsulfonamidato-κN]chloro[(η⁶)-1,3,5-trimethylbenzol]-ruthenium(II)
[N-[(1R,2R und 1S,2S)-2-(amino-κN)-1,2-diphenylethyl]-3-methoxyphenylsulfonamidato-κN]chloro[(η⁶)-1,3,5-trimethylbenzol]-ruthenium(II)
[N-[(1R,2R und 1S,2S)-2-(amino-κN)-1,2-diphenylethyl]-2-methoxyphenylsulfonamidato-κN]chloro[(η⁶)-1,3,5-trimethylbenzol]-ruthenium(II)ato-κN]chloro[(η⁶)-1,3,5-trimethylbenzol]-ruthenium(II)
[N-[(1R,2R und 1S,2S)-2-(amino-κN)-1,2-diphenylethyl]-1-naphtylsulfonamidato-κN]-chloro[(η⁶)-1,3,5-trimethylbenzol]-ruthenium(II)
[N-[(1R,2R und 1S,2S)-2-(amino-κN)-1,2-diphenylethyl]-2-naphtylsulfonamidato-κN]-chloro[(η⁶)-1,3,5-trimethylbenzol]-ruthenium(II)
[N-[(1R,2R und 1S,2S)-2-(amino-κN)-1,2-diphenylethyl]-pentafluorphenylsulfonamidato-κN]chloro[(η⁶)-1,3,5-trimethylbenzol]-ruthenium(II)
[N-[(1R,2R und 1S,2S)-2-(amino-κN)-1,2-diphenylethyl]-methansulfonamidato-κN]-chloro[(η⁶)-1,3,5-trimethylbenzol]-ruthenium(II)
1-[(1R,2R und 1S,2S)-2-(amino-κN)-1,2-diphenylethyl]-trifluormethansulfonamidato-κN]chloro[(η⁶)-1,3,5-trimethylbenzol]-ruthenium(II)
[N-[(1R,2R und 1S,2S)-2-(amino-κN)-1,2-diphenylethyl]-p-tolylsulfonamidato-κN]-chloro[(η⁶)-benzol]-ruthenium(II)
[N-[(1R,2R und 1S,2S)-2-(amino-κN)-1,2-diphenylethyl]-o-tolylsulfonamidato-κN]-chloro[(η⁶)-benzol]-ruthenium(II)
[N-[(1R,2R und 1S,2S)-2-(amino-κN)-1,2-diphenylethyl]-m-tolylsulfonamidato-κN]-chloro[(η⁶)-benzol]-ruthenium(II)
[N-[(1R,2R und 1S,2S)-2-(amino-κN)-1,2-diphenylethyl]-phenylsulfonamidato-κN]-chloro[(η⁶)-benzol]-ruthenium(II)
[N-[(1R,2R und 1S,2S)-2-(amino-κN)-1,2-diphenylethyl]-4-ethylphenylsulfonamidato-κN]chloro[(η⁶)-benzol]-ruthenium(II)
[N-[(1R,2R und 1S,2S)-2-(amino-κN)-1,2-diphenylethyl]-3-ethylphenylsulfonamidato-κN]chloro[(η⁶)-benzol]-ruthenium(II)
[N-[(1R,2R und 1S,2S)-2-(amino-κN)-1,2-diphenylethyl]-2-ethylphenylsulfonamidato-κN]chloro[(η⁶)-benzol]-ruthenium(II)
[N-[(1R,2R und 1S,2S)-2-(amino-κN)-1,2-diphenylethyl]-2,4,6-trimethylphenylsulfonamidato-κN]chloro[(η⁶)-benzol]-ruthenium(II)
[N-[(1R,2R und 1S,2S)-2-(amino-κKN)-1,2-diphenylethyl]-2,4,6-triisopropylphenylsulfonamidato-κN]chloro[(η⁶)-benzol]-ruthenium(II)
[N-[(1R,2R und 1S,2S)-2-(amino-κN)-1,2-diphenylethyl]-4-chlorphenylsulfonamidato-κN]chloro[(η⁶)-benzol]-ruthenium(II)
[N-[(1R,2R und 1S,2S)-2-(amino-κN)-1,2-diphenylethyl]-3-chlorphenylsulfonamidato-κN]chloro[(η⁶)-benzol]-ruthenium(II)
[N-[(1R,2R und 1S,2S)-2-(amino-κN)-1,2-diphenylethyl]-2-chlorphenylsulfonamidato-κN]chloro[(η⁶)-benzol]-ruthenium(II)
[N-[(1R,2R und 1S,2S)-2-(amino-κN)-1,2-diphenylethyl]-4-fluorphenylsulfonamidato-κN]chloro[(η⁶)-benzol]-ruthenium(II)
[N-[(1R,2R und 1S,2S)-2-(amino-κN)-1,2-diphenylethyl]-3-fluorphenylsulfonamidato-κN]chloro[(η⁶)-benzol]-ruthenium(II)
[N-[(1R,2R und 1S,2S)-2-(amino-κN)-1,2-diphenylethyl]-2-fluorphenylsulfonamidato-κN]chloro[(η⁶)-benzol]-ruthenium(II)
[N-[(1R,2R und 1S,2S)-2-(amino-κN)-1,2-diphenylethyl]-4-methoxyphenylsulfonamidato-κN]chloro[(η⁶)-benzol]-ruthenium(II)
[N-[(1R,2R und 1S,2S)-2-(amino-κN)-1,2-diphenylethyl]-3-methoxyphenylsulfonamidato-κN]chloro[(η⁶)-benzol]-ruthenium(II)
[N-[(1R,2R und 1S,2S)-2-(amino-κN)-1,2-diphenylethyl]-2-methoxyphenylsulfonamidato-κN]chloro[(η⁶)-benzol]-ruthenium(II)ato-κN]chloro[(η⁶)-benzol]-ruthenium(II)
[N-[(1R,2R und 1S,2S)-2-(amino-κN)-1,2-diphenylethyl]-1-naphtylsulfonamidato-κN]-chloro[(η⁶)-benzol]-ruthenium(II)
[N-[(1R,2R und 1S,2S)-2-(amino-κN)-1,2-diphenylethyl]-2-naphtylsulfonamidato-κN]chloro[(η⁶)-benzol]-ruthenium(II)
[N-[(1R,2R und 1S,2S)-2-(amino-κN)-1,2-diphenylethyl]-pentafluorphenylsulfonamidato-κN]chloro[(η⁶)-benzol]-ruthenium(II)
[N-[(1R,2R und 1S,2S)-2-(amino-κN)-1,2-diphenylethyl]-methansulfonamidato-κN]-chloro[(η⁶)-benzol]-ruthenium(II)
[N-[(1R,2R und 1S,2S)-2-(amino-κN)-1,2-diphenylethyl]-trifluormethansulfonamidato-κN]chloro[(η⁶)-benzol]-ruthenium(II)
[N-[(1R,2R und 1S,2S)-2-(amino-κN)-cyclohexyl]-p-tolylsulfonamidato-κN]chloro[(η⁶)-benzol]-ruthenium(II)
[N-[(1R,2R und 1S,2S)-2-(amino-κN)-cyclohexyl]-m-tolylsulfonamidato-κN]chloro[(η⁶)-benzol]-ruthenium(II)
[N-[(1R,2R und 1S,2S)-2-(amino-κN)-cyclohexyl]-phenylsulfonamidato-κN]chloro[(η⁶)-benzol]-ruthenium(II)
[N-[(1R,2R und 1S,2S)-2-(amino-κN)-cyclohexyl]-4-ethylphenylsulfonamidato-κN]chloro-[(η⁶)-benzol]-ruthenium(II)
[N-[(1R,2R und 1S,2S)-2-(amino-κN)-cyclohexyl]-2,4,6-trimethylphenylsulfonamidato-κN]chloro[(η⁶)-benzol]-ruthenium(II)
[N-[(1R,2R und 1S,2S)-2-(amino-κN)-cyclohexyl]-4-chlorphenylsulfonamidato-κN]-chloro[(η⁶)-benzol]-ruthenium(II)
[N-[(1R,2R und 1S,2S)-2-(amino-κN)-cyclohexyl]-3-chlorphenylsulfonamidato-κN]-chloro[(η⁶)-benzol]-ruthenium(II)
[N-[(1R,2R und 1S,2S)-2-(amino-κN)-cyclohexyl]-4-fluorphenylsulfonamidato-κN]-chloro[(η⁶)-benzol]-ruthenium(II)
[N-[(1R,2R und 1S,2S)-2-(amino-κN)-cyclohexyl]-3-fluorphenylsulfonamidato-κN]-chloro[(η⁶)-benzol]-ruthenium(II)
[N-[(1R,2R und 1S,2S)-2-(amino-κN)-cyclohexyl]-4-methoxyphenylsulfonamidato-κN]-chloro[(η⁶)-benzol]-ruthenium(II)
[N-[(1R,2R und 1S,2S)-2-(amino-κN)-cyclohexyl]-3-methoxyphenylsulfonamidato-κN]-chloro[(η⁶)-benzol]-ruthenium(II)
[N-[(1R,2R und 1S,2S)-2-(amino-κN)-cyclohexyl]-1-naphtylsulfonamidato-κN]chloro[(η⁶)-benzol]-ruthenium(II)
[N-[(1R,2R und 1S,2S)-2-(amino-κN)-cyclohexyl]-2-naphtylsulfonamidato-κN]chloro[(η⁶)-benzol]-ruthenium(II)
[N-[(1R,2R und 1S,2S)-2-(amino-κN)-cyclohexyl]-methansulfonamidato-κN]chloro[(η⁶)-benzol]-ruthenium(II)
[N-[(1R,2R und 1S,2S)-2-(amino-κN)-cyclohexyl]-trifluormethansulfonamidato-κN]chloro-[(η⁶)-benzol]-ruthenium(II)
[N-[(1R,2R und 1S,2S)-2-(amino-κN)-cyclohexyl]-p-tolylsulfonamidato-κN]chloro[(η⁶)-cumol]-ruthenium(II)
[N-[(1R,2R und 1S,2S)-2-(amino-κN)-cyclohexyl]-m-tolylsulfonamidato-κN]chloro[(η⁶)-cumol]-ruthenium(II)
[N-[(1R,2R und 1S,2S)-2-(amino-κN)-cyclohexyl]-phenylsulfonamidato-κN]chloro[(η⁶)-cumol]-ruthemum(II)
[N-[(1R,2R und 1S,2S)-2-(amino-κN)-cyclohexyl]-4-ethylphenylsulfonamidato-κN]chloro-[(η⁶)-cumol]-ruthenium(II)
[N-[(1R,2R und 1S,2S)-2-(amino-κN)-cyclohexyl]-2,4,6-trimethylphenylsulfonamidato-κN]chloro[(η⁶)-cumol]-ruthenium(II)
[N-[(1R,2R und 1S,2S)-2-(amino-κN)-cyclohexyl]-4-chlorphenylsulfonamidato-κN]-chloro [(η⁶)-cumol]-ruthenium(II)
[N-[(1R,2R und 1S,2S)-2-(amino-κN)-cyclohexyl]-3-chlorphenylsulfonamidato-κN]-chloro[(η⁶)-cumol]-ruthenium(II)
[N-[(1R,2R und 1S,2S)-2-(amino-κN)-cyclohexyl]-4-fluorphenylsulfonamidato-κN]-chloro[(η⁶)-cumol]-ruthenium(II)
[N-[(1R,2R und 1S,2S)-2-(amino-κN)-cyclohexyl]-3-fluorphenylsulfonamidato-κN]-chloro[(η⁶)-cumol]-ruthenium(II)
[N-[(1R,2R und 1S,2S)-2-(amino-κN)-cyclohexyl]-4-methoxyphenylsulfonamidato-κN]-chloro[(η⁶)-cumol]-ruthenium(II)
[N-[(1R,2R und 1S,2S)-2-(amino-κN)-cyclohexyl]-3-methoxyphenylsulfonamidato-κN]-chloro[(η⁶)-cumol]-ruthenium(II)
[N-[(1R,2R und 1S,2S)-2-(amino-κN)-cyclohexyl]-1-naphtylsulfonamidato-κN]chloro[(η⁶)-cumol]-ruthenium(II)
[N-[(1R,2R und 1S,2S)-2-(amino-κN)-cyclohexyl]-2-naphtylsulfonamidato-κN]chloro[(η⁶)-cumol]-ruthenium(II)
[N-[(1R,2R und 1S,2S)-2-(amino-κN)-cyclohexyl]-methansulfonamidato-κN]chloro[(η⁶)-cumol]-ruthenium(II)
[N-[(1R,2R und 1S,2S)-2-(amino-κN)-cyclohexyl]-trifluormethansulfonamidato-κN]-chloro[(η⁶)-cumol]-ruthenium(II)
[N-[(1R,2R und 1S,2S)-2-(amino-κN)-cyclohexyl]-p-tolylsulfonamidato-κN]chloro[(η⁶)-1,3,5-trimethylbenzol]-ruthenium(II)
[N-[(1R,2R und 1S,2S)-2-(amino-κN)-cyclohexyl]-m-tolylsulfonamidato-κN]chloro[(η⁶)-1,3,5-trimethylbenzol]-ruthenium(II)
[N-[(1R,2R und 1S,2S)-2-(amino-κN)-cyclohexyl]-phenylsulfonamidato-κN]chloro[(η⁶)-1,3,5-trimethylbenzol]-ruthenium(II)
[N-[(1R,2R und 1S,2S)-2-(amino-κN)-cyclohexyl]-4-ethylphenylsulfonamidato-κN]-chloro[(η⁶)-1,3,5-trimethylbenzol]-ruthenium(II)
[N-[(1R,2R und 1S,2S)-2-(amino-κN)-cyclohexyl]-2,4,6-trimethylphenylsulfonamidato-κN]chloro[(η⁶)-1,3,5-trimethylbenzol]-ruthenium(II)
[N-[(1R,2R und 1S,2S)-2-(amino-κN)-cyclohexyl]-4-chlorphenylsulfonamidato-κN]-chloro[(η⁶)-1,3,5-trimethylbenzol]-ruthenium(II)
[N-[(1R,2R und 1S,2S)-2-(amino-κN)-cyclohexyl]-3-chlorphenylsulfonamidato-κN]-chloro[(η⁶)-1,3,5-trimethylbenzol]-ruthenium(II)
[N-[(1R,2R und 1S,2S)-2-(amino-κN)-cyclohexyl]-4-fluorphenylsulfonamidato-κN]-chloro[(η⁶)-1,3,5-trimethylbenzol]-ruthenium(II)
[N-[(1R,2R und 1S,2S)-2-(amino-κN)-cyclohexyl]-3-fluorphenylsulfonamidato-κN]-chloro[(η⁶)-1,3,5-trimethylbenzol]-ruthenium(II)
[N-[(1R,2R und 1S,2S)-2-(amino-κN)-cyclohexyl]-4-methoxyphenylsulfonamidato-κN]-chloro[(η⁶)-1,3,5-trimethylbenzol]-ruthenium(II)
[N-[(1R,2R und 1S,2S)-2-(amino-κN)-cyclohexyl]-3-methoxyphenylsulfonamidato-κN]chloro[(η⁶)-1,3,5-trimethylbenzol]-ruthenium(II)
[N-[(1R,2R und 1S,2S)-2-(amino-κN-cyclohexyl]-1-naphtylsulfonamidato-κN]chloro[(η⁶)-1,3,5-trimethylbenzol]-ruthenium(II)
[N-[(1R,2R und 1S,2S)-2-(amino-κN)-cyclohexyl]-2-naphtylsulfonamidato-κN]chloro[(η⁶)-1,3,5-trimethylbenzol]-ruthenium(II)
[N-[(1R,2R und 1S,2S)-2-(amino-κN)-cyclohexyl]-methansulfonamidato-κN]chloro[(η⁶)-1,3,5-trimethylbenzol)-ruthenium(II) und
[N-[(1R,2R und 1S,2S)-2-(amino-κN)-cyclohexyl]-trifluormethansulfonamidato-κN]-chloro[(η⁶)-1,3,5-trimethylbenzol]-ruthenium(II).

Besonders bevorzugte Katalysatoren für Schritt a) sind solche, die Rutheniumkomplexe enthalten, die durch Umsetzung von S,S- oder R,R-N-p-Toluolsulfonyl-1,2-diphenylethylendiamin und Cumoldichlororuthenium-Dimer erhältlich sind.

Gemäß ii) wird in Gegenwart von mindestens einem Amin gearbeitet, das zumindest teilweise in protonierter Form vorliegt.

Weiterhin werden für ii) Ameisensäure, Formiate oder Mischungen davon eingesetzt.

Bevorzugt werden Mischungen von Ameisensäure mit Aminen eingesetzt. Auf diese Weise bilden sich zumindest teilweise die entsprechenden Ammoniumformiate, die analog eingesetzt werden können.

Als Amine sind insbesondere solche der Formel (XV) geeignet,

NR¹⁰R¹¹R¹² (XV)

in der
R¹⁰, R¹¹ und R¹² jeweils unabhängig voneinander für Wasserstoff, C₁-C₈-Alkyl oder Benzyl stehen.

Besonders bevorzugte Amine sind Ammoniak und solche der Formel (XV) in der R , R und R¹² jeweils unabhängig voneinander für C₁-C₈-Alkyl oder Benzyl stehen.

Besonders bevorzugte Amine sind solche der Formel (XV) in der R¹⁰, R¹¹ und R¹² jeweils identisch für Ethyl, n-Butyl oder n-Hexyl stehen, wobei der Einsatz von Triethylamin noch weiter bevorzugt ist.

Das molare Verhältnis von Ameisensäure zu tertiärem Amin kann beispielsweise 1:1 bis 3:1 betragen, bevorzugt ist ein Verhältnis von 1,01:1 bis 1,5:1.

Das molare Verhältnis an Ameisensäure bezogen auf eingesetztes Substrat kann beispielsweise 1:1 bis 3: 1.betragen, bevorzugt sind 1: 1 bis 1,5: 1, besonders bevorzugt 1,02:1 bis 1,1:1.

Schritt a) kann gemäß iii) in Gegenwart oder Abwesenheit, bevorzugt in Gegenwart von organischem Lösungsmittel durchgeführt werden.

Geeignete organische Lösungsmittel sind beispielsweise:

Amide wie z.B. Dimethylformamid, N-Methylpyrrolidinon, gegebenenfalls halogenierte aliphatische oder araliphatische Lösungsmittel mit bis zu 16 Kohlenstoffatomen wie z.B. Toluol, o-, m-, p-Xylol, Chloroform, Dichlormethan, Chlorbenzol, die isomeren Dichlorbenzole, Fluorbenzol, Nitrile wie zum Beispiel Acetonitril, Benzonitril, Dimethylsulfoxid oder Mischungen davon.

Bevorzugte Lösungsmittel sind Acetonitril, N-Methylpyrrolidinon, Chloroform, Dichlormethan, Chlorbenzol, die isomeren Dichlorbenzole, Fluorbenzol oder Mischungen davon, besonders bevorzugt sind Dichlormethan, Acetonitril, N-Methylpyrrolidinon oder Mischungen davon.

Die Reaktionstemperatur kann beispielsweise -10 bis 150°C betragen, bevorzugt sind 20 bis 100°C, besonders bevorzugt 20 bis 80°C.

Die Reaktionszeiten liegen beispielsweise zwischen 0,5 h und 48 h, bevorzugt zwischen 6 und 24 h.

Die molare Menge an Ruthenium kann beispielsweise 0,01 bis 1,0 mol-%, bezogen auf das eingesetzte Substrat betragen, bevorzugt sind 0,02 bis 0,2 mol-%, ganz besonders bevorzugt 0,02 bis 0,1 mol-%.

Es ist vorteilhaft, wenn auch nicht obligatorisch, die Reaktion in einer im Wesentlichen sauerstofffreien Atmosphäre durchzuführen. Im Wesentlichen sauerstofffrei bedeutet dabei beispielsweise einen Gehalt von 0 bis 1 Vol-%, bevorzugt 0 bis 0,1 Vol-% Sauerstoff.

Die Reaktion kann durch die Entfernung von Kohlendioxid, welches während der Reaktion freigesetzt wird, beschleunigt werden. Vorteilhaft und daher von der Erfindung umfasst ist intensives Rühren des Reaktionsgemisches mit einer durchschnittlichen Rührerdrehzahl von beispielsweise 100 bis 3 000 min⁻¹, bevorzugt 500 bis 1 500 min⁻¹. Alternativ oder in Ergänzung dazu kann die Entfernung von Kohlendioxid durch Durchleiten oder Überleiten eines inerten Gasstroms durch oder über die Reaktionsmischung unterstützt werden. Geeignete Gase sind beispielsweise Stickstoff, Edelgase wie z.B. Argon oder Mischungen davon.

Eine besonders bevorzugte Ausführung des Schrittes a) wird nachfolgend beschrieben, ohne jedoch einschränkend zu sein.

In einem Rührkessel wird eine 1:1 Mischung (molar) aus Ameisensäure und Triethylamin durch einfaches Mischen hergestellt und Verbindungen der Formel (II) oder die Verbindungen der Formel (IV) äquimolar oder in geringem Unterschuss zu dieser zweiphasigen Mischung gegeben. Je nach Löslichkeit des Substrates wird eine Menge eines organischen Lösungsmittels zugegeben. Diese Mischung wird durch Durchleiten von Stickstoff inertisiert und die Mischung unter starkem Rühren auf die gewünschte Reaktionstemperatur temperiert.

Zu dieser Mischung wird der Katalysator als Lösung in Dichlormethan in Molverhältnissen gegenüber dem Substrat von beispielsweise 1:500 bis 1:5 000 gegeben und die Reaktionsmischung die gewünschte Zeit gerührt. Der Umsatz wird chromatographisch verfolgt.

Anschließend kann die Reaktionsmischung nach dem Fachmann bekannten Verfahren aufgearbeitet werden. Es hat sich als vorteilhaft erwiesen, zur Aufarbeitung der Reaktionsmischung Lösungsmittel und verdünnte wässrige Salzsäure oder Wasser zuzusetzen. Nach Phasentrennung kann das Produkt aus der organischen Phase entweder destillativ oder durch ein geeignetes Kristallisationsverfahren in an sich bekannter Weise isoliert werden.

Gemäß Schritt a) erhält man enantiomerenangereicherte Verbindungen der Formeln (III) oder (V) mit den oben angegebenen Bedeutungen und Vorzugsbereichen.

Dabei sind je nach Wahl der Konfiguration der Liganden die S- oder R-konfigurierten Produkte erhältlich, wobei sich die Konfigurationsangabe auf das dem Rest Ar benachbarte Kohlenstoffatom bezieht.

Die Verbindungen der Formeln (III) oder (V) können isoliert oder direkt weiter umgesetzt werden. Zur Zwischenisolierung kann die Reaktionsmischung beispielsweise zwischen Wasser und einem mit Wasser wenig mischbaren organischen Lösungsmittel aufgetrennt werden und das gewünschte Produkt in die organische Phase überführt werden. Nach der Entfernung des organischen Lösungsmittels erhält man ein Rohprodukt, das beispielsweise über Kristallisation oder Destillation aufgereinigt werden kann.

Wurden für den Schritt a) Verbindungen der Formel (II) eingesetzt, werden enantiomerenangereicherte Verbindungen der Formel (III) erhalten, die gemäß Schritt b) mit Aminen der Formel (VI) und den dort genannten Bedeutungen und Vorzugsbereichen umgesetzt werden.

Das kann beispielsweise so geschehen, dass die enantiomerenangereicherten Verbindungen der Formel (III) gegebenenfalls in einem Lösungsmittel mit den Aminen der Formel (VI) umgesetzt werden. Eine Übersicht zur Darstellung von Carbonsäureamiden aus Carbonsäuren, Carbonsäureestern, Carbonsäureanhydriden und anderen Carbonsäureamiden bietet Houben-Weyl, 4. Auflage, Band E 5, 941-1010.

Bei flüssigen und gasförmigen Aminen eignet sich die Verwendung von Lösungen der Amine. Beispielsweise können im Fall von Methylamin Lösungen aus Methylamin in Wasser, Methanol oder in Ethanol vorteilhaft für die Umsetzung von Carbonsäureestern der Formel (III) eingesetzt werden. Für die Überführung freier Carbonsäuren der Formel (III) in die Amide der Formel (V) eignen sich beispielsweise Umsetzungen von Aminen der allgemeinen Formel (VI) in Gegenwart von Kupplungsreagenzien wie 2-Halogenpyridinium oder -1,3-thiazolium-Salzen oder in Gegenwart von sauren Kationenaustauschern.

Gemäß Schritt b) werden dann aus enantiomerenangereicherten Verbindungen der Formel (III) enantiomerenangereicherte Verbindungen der Formel (V) erhalten.

Vom Umfang der Erfindung sind auch folgende Verbindungen der Formel (V) umfasst:
(S)-3-Hydroxy-3-(2-thiophen-yl)-N-methylpropionsäureamid, (R)-3-Hydroxy-3-(2-thiophenyl)-N-methylpropionsäüreamid und beliebige Mischungen dieser Verbindungen wie zum Beispiel das Racemat.

Insbesondere sei (S)-3-Hydroxy-3-(2-thiophen-yl)-N-methylpropionsäureamid genannt.

Die enantiomerenangereicherten Verbindungen der Formel (V) können dann zu den enantiomerenangereicherten Verbindungen der Formel (I) reduziert werden. Die Reduktion von Carbonsäureamiden zu den entsprechenden Aminen ist prinzipiell bekannt und in Houben Weyl "Methoden der Organischen Chemie", 4. Auflage, Band E 16 d, 987 -1003 zusammenfassend dargestellt.

Bevorzugt ist die Umsetzung von Verbindungen der Formel (V) mit komplexen Bor- oder Aluminiumhydriden wie z.B. Lithiumaluminiumhydrid, Red-Al® (Natrium-bis-(2-methoxyethoxy)-dihydroaluminat) oder Natriumborhydrid.

Besonders bevorzugt ist die Umsetzung von Verbindungen der Formel (V) mit Lithiumaluminiumhydrid.

Schritt c) wird bevorzugt bei Temperaturen im Bereich von 0 bis 150°C durchgeführt, besonders bevorzugt im Bereich von 50 bis 110°C. Üblicherweise werden die Reduktionen in Ethern als Lösungsmittel durchgeführt, bevorzugt in cyclischen Ethern wie

Tetrahydrofuran oder Dioxan, Reduktionen mit Red-Al ® lassen sich jedoch ebenso in Toluol als Lösungsmittel durchführen.

Auf erfindungsgemäße Weise erhält man die enantiomerenangereicherten Verbindungen der Formel (I), wobei Ar, R¹ und R² die oben genannten Bedeutungen und Vorzugsbereiche besitzen.

Als Einzelverbindungen der Formel (I) seien genannt:
(1S)-3-(Methylamino)-1-(2-thiophen-yl)-1-propanol, (1R)-3-(Methylamino)-1-(2-thiophenyl)-1-propanol, (1S)-3-(Dimethylamino)-1-(2-thiophen-yl)-1-propanol, (1R)-3-(Dimethylamino)-1-(2-thiophen-yl)-1-propanol, (1S)-3-(Methylamino)-1-(phenyl)-1-propanol, (1R)-3-(Methylamino)-1-(phenyl)-1-propanol, (1S)-3-(Methylamino)-1-(4-tolyl)-1-propanol und (1R)-3-(Methylamino)-1-(4-tolyl)-1-propanol.

Die erfindungsgemäß herstellbaren enantiomerenangereicherten Verbindungen der Formel (I) eignen sich insbesondere zur Herstellung von enantiomerenangereicherten Verbindungen der Formel (XVI)

Ar-CH(OR¹⁰)-CH₂-CH₂NR¹R² (XVI)

in der Ar, R¹ und R² die unter der Formel (I) angegebenen Bedeutung und Vorzugsbereiche besitzen und

R¹⁰ für Phenyl, oder Naphthyl steht, das gar nicht, einfach oder mehrfach durch Substituenten substituiert sein kann, die jeweils unabhängig voneinander ausgewählt sind aus der Gruppe Cyano, CO-(C₁-C₁₂-Alkyl), O-(C₁-C₁₂-Alkyl), (C₁-C₁₂-Alkyl), Fluor, Chlor, Brom, C₁-C₁₂-Fluoralkyl, wobei Fluoralkyl für einen einfach, mehrfach oder vollständig durch Fluor substituierten Alkylrest steht.

Bevorzugt steht R¹⁰ für Naphthyl.

Bevorzugte Verbindungen der Formel (XVI) sind:
(S)-N-methyl-3-(1-naphthalenyloxy)-3-(2-thiophen-yl)propylamin und (R)-N-methyl-3-(1-naphthalenyloxy)-3-(2-thiophen-yl)propylamin und Salze von beiden, wobei (S)-N-methyl-3-(1-naphthalenyloxy)-3-(2-thienyl)propylamin besonders bevorzugt ist.

Vom Umfang der Erfindung ist daher auch ein Verfahren umfasst, das als Schritt
d) die Umsetzung von Verbindungen der Formel (I) mit Verbindungen der Formel (XVII) in Gegenwart einer Base umfasst.

In Formel (XVII)

R¹⁰-Hal (XVII)

besitzt R¹⁰ die unter der Formel (XVI) genannte Bedeutung und Vorzugsbereiche und Hal steht für Fluor, Chlor, Brom oder Iod, bevorzugt Fluor.

Als Verbindungen der Formel (XVII) werden bevorzugt 1-Fluornaphthalin und 4-Chlorbenzotrifluorid eingesetzt.

Als Base können solche eingesetzt werden, die die Verbindungen der Formel (I) an der Alkoholfunktion zumindest teilweise deprotonieren können.

Bevorzugte Basen sind Alkalimetallhydroxide und Hydride wie z.B. Natriumhydrid gegebenfalls unter Zusatz von Kaliumbenzoat oder Kaliumacetat, wie in US 5,362,886 beschrieben, Natriumhydroxid und Kaliumhydroxid.

Die Verbindungen der Formeln (I), (V) und (XVI) eignen sich insbesonders zur Herstellung von Pharmazeutika wie vorzugsweise Serotonin- oder Noradrenalin-Aufnahmeinhibitoren.

Das erfindungsgemäße Verfahren hat den Vorteil, dass ausgehend von einfach verfügbaren Edukten die Synthese von enantiomerenangereicherten 1-Aryl-3-amino-propanolen der Formel (I) und deren Folgeprodukten in hohen Gesamtausbeuten, hohen Enantiomerenüberschüssen und hohen Reinheiten im technischen Maßstab möglich ist.

Ein weiterer gegenstand der Erfindung sind (S)-3-Hydroxy-3-(2-thiophen-yl)-N-methylpropionsäureamid und (R)-3-Hydroxy-3-(2-thiophen-yl)-N-methylpropionsäureamid sowie die racemischen und beliebigen Mischungen dieser Verbindungen.

### Beispiele:

### Beispiel 1:

In einen 2-Liter-Kolben wurden 180,8 g Natriummethylat und 1 500 ml Toluol auf 100°C erhitzt und anschließend innerhalb von 4 Stunden eine Lösung aus 257 g 2-Acetylthiophen in 510 ml Dimethylcarbonat zugetropft. Das in der Reaktion entstandene Methanol wurde dabei als Azeotrop abdestilliert. In einem 4-Liter Kolben wurden 120 ml konz. Schwefelsäure in 900 g Eis vorgelegt und das abgekühlte Reaktionsgemisch so zugegeben, dass 40°C nicht überschritten wurden. Es wurde nachgerührt und der pH-Wert auf pH 1 eingestellt. Die Phasen wurden getrennt und die organische Phase dreimal mit wäßriger Natriumsulfatlösung ausgeschüttelt und anschließend im Vakuum eingeengt. Die Vakuumdestillation des Rohprodukts lieferte 278 g β-Oxo-(2-thiophen)-propionsäuremethylester als transparente, leicht gelbliche Flüssigkeit (98 % rein nach GC, 74% d. Th.).

### Beispiel 2:

### Methyl (3S)-3-hydroxy-3-(2-thiophen-yl)propanoat

In einem Schlenkgefäß wird die Katalysatorlösung durch Einwaage von 314 mg (2.03 Äquiv.) S,S-TsDPEN und 263 mg [(Cumol)RuCl₂]₂ in 10 ml CH₂Cl₂ sowie Versetzen mit 0,3 ml (2 Äquiv.) Et₃N und Rühren dieses Gemisches für 15 min bei Raumtemperatur hergestellt.

In einem 1-Liter-Mehrhalskolben mit Begasungsrührer, Rückflußkühler und Innenthermometer wird eine HCOOH/Et₃N-Mischung (Molverhältnis 1:1) durch langsames Zutropfen innerhalb von 20 min von 41 ml HCOOH zu 152 ml Et₃N unter Rührung und Eiskühlung hergestellt. Zu diesem zweiphasigen Gemisch werden 190 g Methyl-2-Oxo-3-(2-thiophen-nyl)propionat gegeben, die homogene gelbe Lösung mit 0,1 1 Dichlormethan versetzt und die Gesamtmischung durch Durchleiten von Argon für 20 min entgast. Es wird auf 36°C temperiert und unter starkem Rühren die dunkelrote Katalysatorlösung auf einmal zum Reaktionsansatz gegeben. Es wird unter Durchleiten von Argon durch den Reaktionsansatz für 14 h mit 800 U/min gerührt.

Nach Verdünnen mit 0,3 1 1N HCl und 0,31 CH₂Cl₂ und Phasentrennung wird die H₂O Phase noch 2 x mit CH₂Cl₂ extrahiert, die vereinigten org. Phasen mit 150 ml NaCl-Lösung gewaschen, über MgSO₄ getrocknet, filtriert und das Lösungsmittel entfernt. Das Rohprodukt wird ohne Aufreinigung in die nächste Stufe eingesetzt; 100 % Umsatz.

Die Umsatz- und Enantiomeren-Analytik erfolgte gaschromatographisch auf einem HP Gaschromatographen unter Verwendung einer IVADEX Kapillarsäure (12,5 m, 0,3 µm Schichtdicke unter Verwendung individueller Temperaturprogramme). 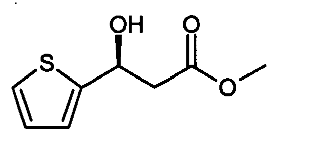 ¹H-NMR (d1-Chloroform, 400 MHz): δ = 7.23 (m, 1H, Ar-H), 6.95 (m, 2H, Ar-H), 5.36 (dd, 1H, CHOH), 3.71 (s, 3H, OCH₃), 2.86 (m, 2H, CHH) ppm.
¹³C-NMR (d1-Chloroform, 100 MHz): δ = 185.3 (C=O), 146.8 (C, Ar), 127.1 (CH, Ar), 125.3 (CH, Ar), 124.1 (CH, Ar), 66.9 (CHOH), 52.4 (CH3), 43.5 (CH2) ppm.

Chiral-GC: 14.05, 14.41 min. ee = 98,2 %.

### Beispiel 3:

115 g Methyl (3S)-3-hydroxy-3-(2-thiophen-nyl)propanoat werden vorgelegt und mit 618 ml einer 2-molaren methanolischen Methylamin-Lösung versetzt. Diese Mischung wird für 4 h bei 60°C gerührt, abgekühlt und anschließend im Vakuum eingeengt. So erhält man 118 g N-Methyl-(3S)-3-hydroxy-3-(2-thiophen-yl)propanamid (Reinheit 86 %; 88 % d.Th.). Das Rohprodukt kann in die nächste Stufe eingesetzt werden oder aber aus Methylenchlorid und Hexan umkristallisiert werden. Dies lieferte 93 g N-Methyl-(3S)-3-hydroxy-3-(2-thiophenyl)-propanamid (76 % d. Th.) als weiße Kristalle. Alternativ dazu kann auch die Reinigung durch Destillation erfolgen.

Die Umsatz- und Enantiomeren-Analytik erfolgte gaschromatographisch auf einem HP Gaschromatographen unter Verwendung einer IVADEX Kapillarsäure (12,5 m, 0,3 µm Schichtdicke unter Verwendung individueller Temperaturprogramme).

### Beispiel 4:

1 728 ml trockenes Tetrahydrofuran werden mit 52 g Lithiumaluminiumhydrid vorgelegt und auf Rückfluss erhitzt. Gleichzeitig wird damit begonnen, 86,4 g N-Methyl-(3S)-3-hydroxy-3-(2-thiophen-yl)propanamid in 692 g Tetrahydrofuran gelöst zuzutropfen. Nachdem alles zugetropft ist, wird über Nacht bei Rückfluss nachgerührt. Anschließend wird auf Raumtemperatur abgekühlt und es werden 1 037 ml Wasser vorsichtig zugetropft. Dann wurden 173 ml einer 10 %igen Natriumhydroxidlösung zugetropft und die Lösung filtriert. Das Lösungsmittel wurde im Vakuum entfernt. Die Rohlösung wurde mit 346 ml 1 N Natronlauge versetzt und 3-mal mit je 345 ml Toluol extrahiert. Die organischen Phasen werden vereinigt und die flüchtigen Bestandteile im Vakuum entfernt. So erhielt man 76 g (1S)-3-(methylamino)-1-(2-thiophen-yl)-1-propanol (84 % Reinheit, 80 % d.Th.).

## Patentansprüche

1. Verfahren zur Herstellung von enantiomerenangereicherten Verbindungen der Formel (I),
Ar-CH(OH)-CH₂-CH₂-NR¹R² (I)
in der
Ar für einen substituierten oder unsubstituierten 2-Thiophen-yl-rest oder 3-Thiophen-ylrest und
R¹ und R² jeweils unabhängig voneinander für Wasserstoff, C₁-C₂₀-Alkyl, C₄-C₁₄-Aryl oder C₅-C₁₅-Arylalkyl stehen oder die beiden Reste R¹ und R² zusammen für C₃-C₁₂-Alkylen stehen, **dadurch gekennzeichnet, dass**
a) Verbindungen der Formel (II) in enantiomerenangereicherte Verbindungen der Formel (III) oder Verbindungen der Formel (IV) in enantiomerenangereicherte Verbindungen der Formel (V) überführt werden
Ar-CO-CH₂-COOR³ (II)
Ar-CH(OH)-CH₂-COOR³ (III)
Ar-CO-CH₂-CONR¹R² (IV)
Ar-CH(OH)-CH₂-CONR¹R² (V),
wobei jeweils
Ar die unter der Formel (I) genannte Bedeutung besitzt und
R¹ und R² die unter der Formel (I) genannte Bedeutung besitzen und
R³ für Wasserstoff, C₁-C₂₀-Alkyl, C₄-C₁₄ -Aryl oder C₅-C₁₅-Arylalkyl steht,
und wobei die Umsetzung
i) in Gegenwart eines Rutheniumkomplexe-enthaltenden Katalysators ,
wobei die Rutheniumkomplexe durch Umsetzung von Verbindungen der Formel (XII) mit Verbindungen der Formel (XIII) erhältlich sind, oder Komplexe der Formel (XIV), wobei in den Verbindungen der Formel (XII)
[RuX₂(Aren)]₂ (XII)
Aren steht für eine koordinierte aromatische Verbindung mit 6 bis 12 Ringkohlenstoffatomen, die weiterhin mit bis zu 6 Resten substituiert sein kann, die jeweils unabhängig voneinander ausgewählt sind aus der Gruppe C₁-C₈-Alkyl, Benzyl und Phenyl und
X für Chlor, Brom oder Iod steht,
und wobei in der Formel (XIII) 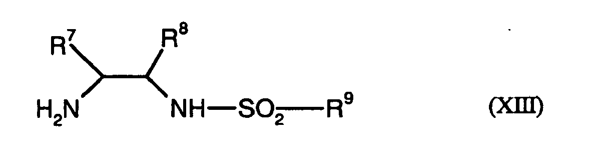 R⁷ und R⁸ jeweils unabhängig voneinander für C₁-C₂₀-Alkyl, C₄-C₁₅-Aryl oder C₅-C₁₆-Arylalkyl oder R⁷ und R⁸ zusammen für einen geradkettigen oder verzweigten C₃-C₁₂-Alkylen-Rest stehen und
R⁹ für C₁-C₂₀-Alkyl, C₁-C₂₀-Fluoralkyl oder C₄-C₁₅-Aryl steht,
und wobei in der Formel (XIV)
[RuX₂(aren){(XIII)}] (XIV)
Aren und X jeweils die unter Formel (XII) angegebene Bedeutung besitzen und (XIII) für Verbindungen der Formel (XIII) mit der dort angegebenen Bedeutung steht,
ii) in Gegenwart von mindestes einem Amin, das zumindest teilweise in protonieter Form vorliegt,
iii) mit Ameisensäure, Formiaten oder Mischungen davon
erfolgt und
b) für den Fall, dass für Schritt a) Verbindungen der Formel (II) eingesetzt wurden, die enantiomerenangereicherten Verbindungen der Formel (III) mit Aminen der Formel (VI)
HNR¹R² (VI),
in der R¹ und R² die unter der Formel (I) genannte Bedeutung besitzen,
zu enantiomerenangereicherten Verbindungen der Formel (V) mit der oben genannten Bedeutung umgesetzt werden und
c) die enantiomerenangereicherten Verbindungen der Formel (V) durch Reduktion in enantiomerenangereicherte Verbindungen der Formel (I) mit der oben genannten Bedeutung überführt werden.

2. Verfahren nach Anspruch 1, **dadurch gekennzeichnet, dass**
d) die enantiomerenangereicherten Verbindungen der Formel (I) in Gegenwart von Base mit Verbindungen der Formel (XVII)
R¹⁰-Hal (XVII),
in der
R¹⁰ für Phenyl oder Naphthyl steht, das gar nicht, einfach oder mehrfach durch Substituenten substituiert sein kann, die jeweils unabhängig voneinander ausgewählt sind aus der Gruppe Cyano, CO-(C₁-C₁₂-Alkyl), O-(C₁-C₁₂-Alkyl), (C₁-C₁₂-Alkyl)Fluor, Chlor, Brom, C₁-C₁₂-Fluoralkyl, wobei Fluoralkyl für einen einfach, mehrfach oder vollständig durch Fluor substituierten Alkylrest steht und
Hal für Fluor, Chlor, Brom oder Iod steht
zu Verbindungen der Formel (XVI)
Ar-CH(OR¹⁰)-CH₂-CH₂-NR¹R² (XVI)
umgesetzt werden,
in der Ar, R¹,R² und R¹⁰ die oben genannten Bedeutungen besitzen.

3. Verfahren nach einem oder mehreren der Ansprüche 1 bis 2, **dadurch gekennzeichnet, dass** im Schritt a) Verbindungen der Formel (II) mit der in Anspruch 1 genannten Bedeutung eingesetzt werden.

4. Verfahren nach Anspruch 3, **dadurch gekennzeichnet, dass** als Verbindungen der Formel (II) Methyl-3-oxo-3-(2-thiophen-yl)propanoat, Ethyl-3-oxo-3-(2-thiophenyl)propanoat, Isopropyl-3-oxo-3-(2-thiophen-yl)propanoat, tert.-Butyl-3-oxo-3-(2-thiophen-yl)-propanoat, 2-Ethylhexyl-3-oxo-3-(2-thiophen-yl)propanoat eingesetzt werden.

5. Verfahren nach einem oder mehreren der Ansprüche 1 bis 4, **dadurch gekennzeichnet, dass** die Verbindungen der Formel (IV) durch Umsetzung von Verbindungen der Formel (IX)
Ar-CO-CH₃ (IX),
in der Ar die unter der Formel (I) genannte Bedeutung und Vorzugsbereiche besitzt, mit Verbindungen der Formel (XI)
R³-O-CO-NR¹R² (XI)
erhalten wurden,
in denen die Reste R¹, R² und R³ jeweils unabhängig voneinander die gleiche Bedeutung und Vorzugsbereiche besitzen, die unter den Formeln (II) und (IV) angegeben wurden.

6. Verfahren nach einem oder mehreren der Ansprüche 1 bis 5, **dadurch gekennzeichnet, dass** als Rutheniumkomplexe solche verwendet werden, die durch Umsetzung von S,S- oder R,R-N-p-Toluolsulfonyl-1,2-diphenylethylendiamin und Cumoldichlororuthenium-Dimer erhältlich sind.

7. Verfahren nach einem oder mehreren der Ansprüche 1 bis 5, **dadurch gekennzeichnet, dass** Mischungen von Ameisensäure und Triethylamin verwendet werden.

8. Verfahren nach einem oder mehreren der Ansprüche 1 bis 7, **dadurch gekennzeichnet, dass** die Reaktionstemperatur -10 bis 150°C beträgt.

9. Verfahren nach einem oder mehreren der Ansprüche 1 bis 8, **dadurch gekennzeichnet, dass** die molare Menge an Ruthenium 0,01 bis 1,0 mol-% bezogen auf das eingesetzte Substrat beträgt.

10. Verfahren nach einem oder mehreren der Ansprüche 6 bis 11, **dadurch gekennzeichnet, dass** die Reduktion im Schritt c) mit Lithiumaluminiumhydrid durchgeführt wird.

11. (S)-3-Hydroxy-3-(2-thiophen-yl)-N-methylpropionsäureamid, (R)-3-Hydroxy-3-(2-thiophen-yl)-N-methylpropionsäureamid sowie die racemische und beliebige Mischungen dieser Verbindungen.

## Claims

1. Process for preparing enantiomerically enriched compounds of the formula (I)
Ar-CH (OH) -CH₂-CH₂-NR¹R² (I)
where
Ar is a substituted or unsubstituted 2-thiophenyl radical or 3-thiophenyl radical and
R¹ and R² are each independently hydrogen, C₁-C₂₀-alkyl, C₄-C₁₄-aryl or C₅-C₁₅-arylalkyl, or the two R¹ and R² radicals together are C₃-C₁₂-alkylene, **characterized in that**
a) compounds of the formula (II) are converted to enantiomerically enriched compounds of the formula (III) or compounds of the formula (IV) are converted to enantiomerically enriched compounds of the formula (V)
Ar-CO-CH₂COOR³ (II)
Ar-CH(OH)-CH₂-COOR³ (III)
Ar-CO-CH₂CONR¹R² (IV)
Ar-CH(OH)-CH₂-CONR¹R² (V)
where, in each case,
Ar is as defined under the formula (I) and
R¹ and R² are each as defined under the formula (I) and
R³ is hydrogen, C₁-C₂₀-alkyl, C₄-C₁₄-aryl or C₅-C₁₅-arylalkyl,
and where the reaction is effected
i) in the presence of a catalyst comprising ruthenium complexes,
the ruthenium complexes being obtainable by reacting compounds of the formula (XII) with compounds of the formula (XIII), or complexes of the formula (XIV) where, in the compounds of the formula (XII)
[RuX₂(arene)]₂ (XII),
arene is a coordinated aromatic compound having 6 to 12 ring carbon atoms which may be further substituted by up to 6 radicals, each of which is independently selected from the group of C₁-C₈-alkyl, benzyl and phenyl and
X is chlorine, bromine or iodine,
and where, in the formula (XIII) 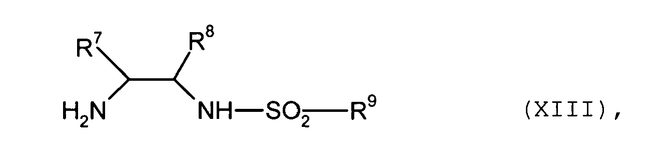 R⁷ and R⁸ are each independently C₁-C₂₀-alkyl, C₄-C₁₅-aryl or C₅-C₁₆-arylalkyl, or R⁷ and R⁸ together are a straight-chain or branched C₃-C₁₂-alkylene radical, and
R⁹ is C₁-C₂₀-alkyl, C₁-C₂₀-fluoroalkyl or C₄-C₁₅-aryl
and where, in the formula (XIV)
[RuX₂(arene){(XIII)}] (XIV),
arene and X are each as defined under formula (XII) and (XIII) represents compounds of the formula (XIII) as defined there,
ii) in the presence of at least one amine which is present is present at least partly in protonated form,
iii) with formic acid, formates or mixtures thereof
and
b) in the case that compounds of the formula (II) have been used for step a), the enantiomerically enriched compounds of the formula (III) are reacted with amines of the formula (VI)
HNR¹R² (VI)
where R¹ and R² are each as defined under the formula (I),
to give enantiomerically enriched compounds of the formula (V) as defined above and
c) the enantiomerically enriched compounds of the formula (V) are converted by reduction to enantiomerically enriched compounds of the formula (I) as defined above.

2. Process according to Claim 1, **characterized in that**
d) the enantiomerically enriched compounds of the formula (I) are reacted in the presence of a base with compounds of the formula (XVII)
R¹⁰-Hal (XVII)
where
R¹⁰ is phenyl or naphthyl which is not at all, singly or multiply substituted by substituents which are each independently selected from the group of cyano, CO- (C₁-C₁₂-alkyl) , O- (C₁-C₁₂-alkyl) , (C₁-C₁₂-alkyl) fluorine, chlorine, bromine, C₁-C₁₂-fluoroalkyl, where fluoroalkyl is a singly, multiply or fully fluorine-substituted alkyl radical and
Hal is fluorine, chlorine, bromine or iodine
to give compounds of the formula (XVI)
Ar-CH (OR¹⁰) -CH₂-CH₂-NR¹R² (XVI)
where Ar, R¹, R² and R¹⁰ are each as defined above.

3. Process according to one or more of Claims 1 to 2, **characterized in that** compounds of the formula (II) as defined in Claim 1 are used in step a).

4. Process according to Claim 3, **characterized in that** the compounds of the formula (II) used are methyl 3-oxo-3-(2-thiophenyl)propanoate, ethyl 3-oxo-3-(2-thiophenyl)propanoate, isopropyl 3-oxo-3-(2-thiophenyl)propanoate, tert-butyl 3-oxo-3-(2-thiophenyl)propanoate, 2-ethylhexyl 3-oxo-3-(2-thiophenyl)propanoate.

5. Process according to one or more of Claims 1 to 4, **characterized in that** the compounds of the formula (IV) have been obtained by reacting compounds of the formula (IX)
Ar-CO-CH₃ (IX)
where Ar has the definition and areas of preference specified under the formula (I) with compounds of the formula (XI)
R³-O-CO-NR¹R² (XI)
where the R¹, R² and R³ radicals each independently have the same definition and areas of preference as stated under the formulae (II) and (IV).

6. Process according to one or more of Claims 1 to 5, **characterized in that** the ruthenium complexes used are those which are obtainable by reacting S,S-or R,R-N-p-toluenesulphonyl-1,2-diphenylethylenediamine and (cumene)dichlororuthenium dimer.

7. Process according to one or more of Claims 1 to 5, **characterized in that** mixtures of formic acid and triethylamine are used.

8. Process according to one or more of Claims 1 to 7, **characterized in that** the reaction temperature is -10 to 150°C.

9. Process according to one or more of Claims 1 to 8, **characterized in that** the molar amount of ruthenium is 0.01 to 1.0 mol%, based on the substrate used.

10. Process according to one or more of Claims 6 to 11, **characterized in that** the reduction in step c) is carried out with lithium aluminium hydride.

11. (S)-3-Hydroxy-3-(2-thiophen-yl)-N-methylpropionamide, (R)-3-hydroxy-3-(2-thiophenyl)-N-methylpropionamide, and the racemic and any desired mixtures of these compounds.

## Revendications

1. Procédé pour la préparation de composés enrichis en énantiomères de formule (I),
Ar-CH (OH) -CH₂-CH₂-NR¹R² (I)
dans laquelle
Ar représente un radical 2-thiophényle ou 3-thiophényle substitué ou non substitué et
R¹ et R² représentent, à chaque fois indépendamment l'un de l'autre, hydrogène, C₁-C₂₀-alkyle, C₄-C₁₄-aryle ou C₅-C₁₅-arylalkyle ou les deux radicaux R¹ et R² représentent ensemble C₃-C₁₂-alkylène,
**caractérisé en ce que**
a) des composés de formule (II) sont transformés en composés enrichis en énantiomères de formule (III) ou des composés de formule (IV) sont transformés en composés enrichis en énantiomères de formule (V)
Ar-CO-CH₂-COOR³ (II)
Ar-CH(OH)-CH₂-COOR³ (III)
Ar-CO-CH₂-CONR¹R² (IV)
Ar-CH (OH) -CH₂-CONR¹R² (V)
où, à chaque fois
Ar présente la signification mentionnée sous la formule (I) et
R¹ et R² présentent la signification mentionnée sous la formule (I) et
R³ représente hydrogène, C₁-C₂₀-alkyle, C₄-C₁₄-aryle ou C₅-C₁₅-arylalkyle,
où la transformation est réalisée
i) en présence d'un catalyseur contenant des complexes de ruthénium,
où les complexes de ruthénium peuvent être obtenus par transformation de composés de formule (XII) avec des composés de formule, (XIII), ou des complexes de formule (XIV), où, dans les composés de formule (XII)
[RuX₂(Arène)]₂ (XII)
Arène représente un composé aromatique coordiné comprenant 6 à 12 atomes de carbone de cycle, qui peut en outre être substitué par jusqu'à 6 radicaux, qui sont à chaque fois choisis indépendamment l'un de l'autre dans le groupe formé par C₁-C₈-alkyle, benzyle et phényle et
X représente chlore, brome ou iode,
et où, dans la formule (XIII) 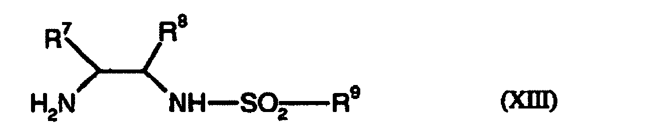
R⁷et R⁸ représentent, à chaque fois indépendamment l'un de l'autre, C₁-C₂₀-alkyle, C₄₋C₁₅-aryle ou C₅₋C₁₆-arylalkyle ou R⁷ et R⁸ représentent ensemble un radical C₃-C₁₂-alkylène linéaire ou ramifié et
R⁹ représente C₁-C₂₀-alkyle, C₁-C₂₀-fluoroalkyle ou C₄-C₁₅-aryle,
et où, dans la formule (XIV)
[RuX₂ (arène){(XIII)}] (XIV)
Arène et X présentent à chaque fois la signification indiquée sous la formule (XII) et (XIII) représente des composés de formule (XIII) avec la signification qui y est indiquée,
ii) en présence d'au moins une amine, qui se trouve au moins partiellement sous forme protonée,
iii) avec de l'acide formique, des formiates ou leurs mélanges
et
b) dans le cas où on utiliserait pour l'étape a) des composés de formule (II), les composés enrichis en énantiomères de formule (III) sont transformés avec des amines de formule (VI)
HNR¹R² (VI),
dans laquelle R¹ et R² présentent la signification mentionnée sous la formule (I),
en composés enrichis en énantiomères de formule (V) avec la signification susmentionnée et
c) on transforme les composés enrichis en énantiomères de formule (V) par réduction en composés enrichis en énantiomères de formule (I) avec la signification susmentionnée.

2. Procédé selon la revendication 1, **caractérisé en ce que**
d) les composés enrichis en énantiomères de formule (I) sont transformés en présence de base avec des composés de formule (XVII)
R¹⁰-Hal (XVII) ,
dans laquelle
R¹⁰ représente phényle ou naphtyle, qui peut être non substitué, monosubstitué ou polysubstitué par des substituants qui sont choisis, à chaque fois indépendamment l'un de l'autre, dans le groupe formé par cyano, CO-(C₁-C₁₂-alkyle), O- (C₁-C₁₂-alkyle) , (C₁-C₁₂-alkyle) , fluor, chlore, brome, C₁-C₁₂-fluoroalkyle, où fluoroalkyle représente un radical alkyle monosubstitué, polysubstitué ou totalement substitué par fluor et
Hal représente fluor, chlore, brome ou iode,
en composés de formule (XVI)
Ar-CH (OR¹⁰)-CH₂-CH₂-NR¹R² (XVI)
où Ar, R¹, R² et R¹⁰ présentent les significations susmentionnées.

3. Procédé selon l'une ou plusieurs des revendications 1 à 2, **caractérisé en ce qu'**on utilise dans l'étape a) des composés de formule (II) présentant la signification mentionnée dans la revendication 1.

4. Procédé selon la revendication 3, **caractérisé en ce qu'**on utilise comme composés de formule (II) le propanoate de méthyl-3-oxo-3-(2-thiophényle), le propanoate d'éthyl-3-oxo-3-(2-thiophényle), le propanoate d'isopropyl-3-oxo-3-(2-thiophényle), le propanoate de tert-butyl-3-oxo-3-(2-thiophényle), le propanoate de 2-éthylhexyl-3-oxo-3-(2-thiophényle).

5. Procédé selon l'une ou plusieurs des revendications 1 à 4, **caractérisé en ce que** les composés de formule (IV) sont obtenus par transformation de composés de formule (IX)
Ar-CO-CH₃ (IX),
dans laquelle Ar présente la signification et les plages préférentielles mentionnée(s) sous la formule (I) avec des composés de formule (XI)
R³-O-CO-NR¹R² (XI)
dans laquelle les radicaux R¹, R² et R³ présentent, à chaque fois indépendamment l'un de l'autre, la même signification et les mêmes plages préférentielles que celle(s) indiquée(s) dans les formules (II) et (IV).

6. Procédé selon l'une ou plusieurs des revendications 1 à 5, **caractérisé en ce qu'**on utilise comme complexes du ruthénium ceux qui peuvent être obtenus par transformation de S,S-N-p-toluènesulfonyl-1,2-diphényléthylènediamine ou de R,R-N-p-toluènesulfonyl-1,2-diphényléthylènediamine et de dimère de cumènedichlororuthénium.

7. Procédé selon l'une ou plusieurs des revendications 1 à 5, **caractérisé en ce qu'**on utilise des mélanges d'acide formique et de triéthylamine.

8. Procédé selon l'une ou plusieurs des revendications 1 à 7, **caractérisé en ce que** la température de réaction est de -10°C à 150°C.

9. Procédé selon l'une ou plusieurs des revendications 1 à 8, **caractérisé en ce que** la quantité molaire de ruthénium est de 0,01 à 1,0% en mole par rapport au substrat utilisé.

10. Procédé selon l'une ou plusieurs des revendications 6 à 11, **caractérisé en ce que** la réduction dans l'étape c) est réalisée avec de l'hydrure de lithiumaluminium.

11. Amide de l'acide (S)-3-hydroxy-3-(2-thiophényl)-N-méthylpropionique, amide de l'acide (R)-3-hydroxy-3-(2-thiophényl)-N-méthylpropionique ainsi que les mélanges racémiques et quelconques de ces composés.
